# EUROPEAN PATENT SPECIFICATION

(11) **EP 0 389 079 B1**
(45) Date of publication and mention of the grant of the patent: **19.04.1995**
(21) Application number: 90250078.4
(22) Date of filing: 21.03.1990
(51) Int. Cl.: C08G 69/10, A61K 31/785

(54) **Polyamides bearing functionalized side chains useful as water soluble hypolipidemic agents**
Polyamide mit funktionalisierten Seitenketten zur Verwendung als wasserlösliche hypolipidemische Agentien
Polyamides portant des chaînes latérales fonctionnalisées utilisables comme agents hypolipidémiques solubles dans l'eau

(30) Priority: 23.03.1989 US 328014
(43) Date of publication of application: 26.09.1990
(73) Proprietor: BERLEX LABORATORIES, INC., Wayne New Jersey 07470 (US)
(72) Inventor: Caldwell, Walton Bernard, Cranbury, NJ 08512 (US); Erhardt, Paul William, Long Valley, NJ 07853 (US); Lumma, William Carl, Jr., Pennsburg, PA 18073 (US); Phillips, Gary Bruce, Wharton, NJ 07885 (US); Shaw, Kenneth Jay, Califon, NJ. 07830 (US); Taggart, William Vroom, Quakertown, New Jersey 08868 (US); Vennepalli, Bhaskar Rao, Fairport, NY 14450 (US)
(74) Representative: Fischer, Klaus Dieter

(56) References cited:
- EP-A- 0 261 017
- WO-A-87/03891
- DE-A- 2 032 470
- GB-A- 2 174 097

## Description

### Field of Invention

Known non-systemic plasma cholesterol lowering agents such as cholestyramine and colestipol are administered as sequestering agents to bind bile acids in the intestinal tract. These form complexes which are excreted in the feces whereby the bile acids which would otherwise be reabsorbed and returned to the liver are removed from the body. As a result, the enterohepatic cycle is interrupted which leads to an increased metabolism of cholesterol to bile acids with a resultant decrease in plasma cholesterol levels. Both cholestyramine and colestipol are water-insoluble resins which in order to be efficacious must be taken in large bulk. Due to this dosing regimen and certain untoward side effects, patient compliance is a problem.

It is the object of this invention to provide certain polyamides which are non-systemic, water soluble polymers which are hypolipidemic agents, that is they are useful in treating hyperlipidemia wherein plasma cholesterol and/or triglyceride levels are excessive. In view of their nature, it is expected these compounds will increase patient compliance for the treatment of hyperlipidemia.

This invention relates to these water soluble polymers which are derivatives of polyanhydroaspartic acid, to their pharmaceutically acceptable salts and their pharmaceutically acceptable formulations.

### General Description Of The Invention

### Composition-Of-Matter Aspect

This invention relates to novel and known polyamides bearing functionalized side chains which are useful as non-systemic water soluble hypolipidemic agents.

The water soluble hypolipidemic compounds of this invention are of the following Formula II: 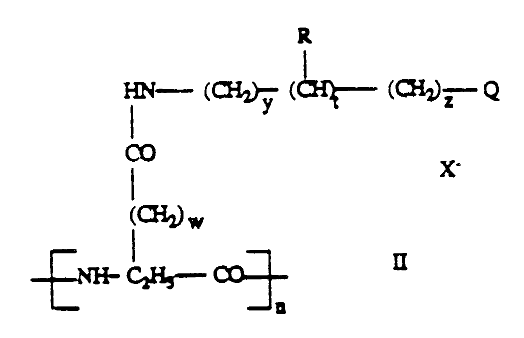
wherein
- w: is the integer 0 or 1;
- y: is the integer 1-6;
- z: is the integer 0-3;
- t: is the integer 0 or 1;
- Q: is

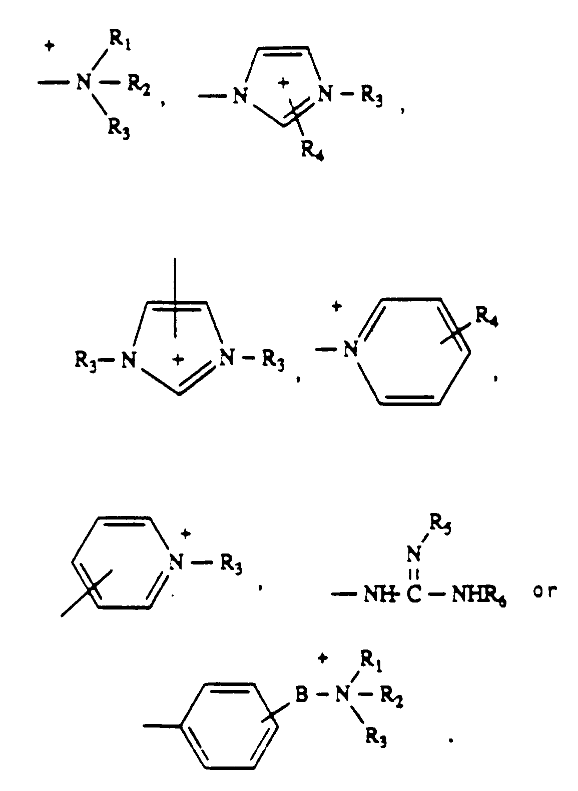

R is hydrogen, C₁-C₄ straight or branched alkyl, phenyl or when taken together with R₁ forms a saturated monoheterocyclic ring of from 5 to 6 members.

R₁, R₂ are the same or independently C₁-C₁₀ straight or branched chain alkyl which may be substituted by up to 3 substituents selected from 1 to 2 hydroxyl groups and one 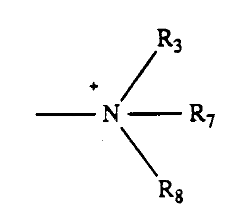
group, or when taken together form a saturated heterocyclic ring of from 5 to 6 members which may contain a 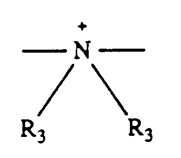
or -O- linkage;
R₃ is methyl, ethyl, benzyl which may be substituted by up to 3 substituents selected from hydroxyl, halogen, methoxy and C₁-C₆ straight or branched chain alkyl, 1-naphthyl or 2-naphthyl.

R₄ is a C₁-C₆ straight or branched chain alkyl which may be substituted by one hydroxyl group.

R₅, R₆ are the same or independently hydrogen, C₁-C₄ straight or branched chain alkyl or when taken together form a diheterocyclic ring of from 5 to 6 members.

R₇, R₈ are the same or independently C₁-C₆ straight or branched chain alkyl which may be substituted by one hydroxyl group or when taken together with an -O- linkage form the morpholine moiety.
- B: is a direct link or a C₁-C₄ straight or branched chain alkyl;
- n: is an integer in the range from 50 to 500; and
- X⁻: is an anion forming a pharmaceutically acceptable salt.

Preferred compounds of the foregoing are those wherein w is the integer 0.

More preferred compounds are those wherein w is the integer 0 and n is in the range of 100 to 500.

Most preferred compounds are those wherein
a) w is the integer 0,
b) n is in the range of 100 to 500,
c) R is H, y is the integer 1, t is the integer 1 and z is 0 or 1.

Particularly preferred compounds are those wherein
a) w is the integer 0,
b) n is in the range of 100 to 500,
c) R is H, y is the integer 1, t is the integer 1 and z is 0 or 1, and
d) R₃ is methyl or benzyl
The foregoing compounds of Formula II which compounds are useful as hypolipidemic agents are inclusive of novel and known compounds. Said novel compounds of the invention are those defined by the following Formula I: 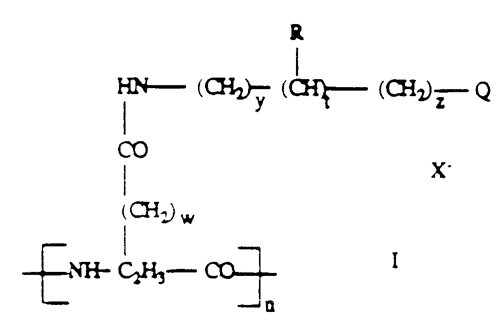
wherein
- w: is the integer 0 or 1;
- y: is the integer 1-6;
- z: is the integer 0-3;
- t: is the integer 0 or 1;
- Q: is 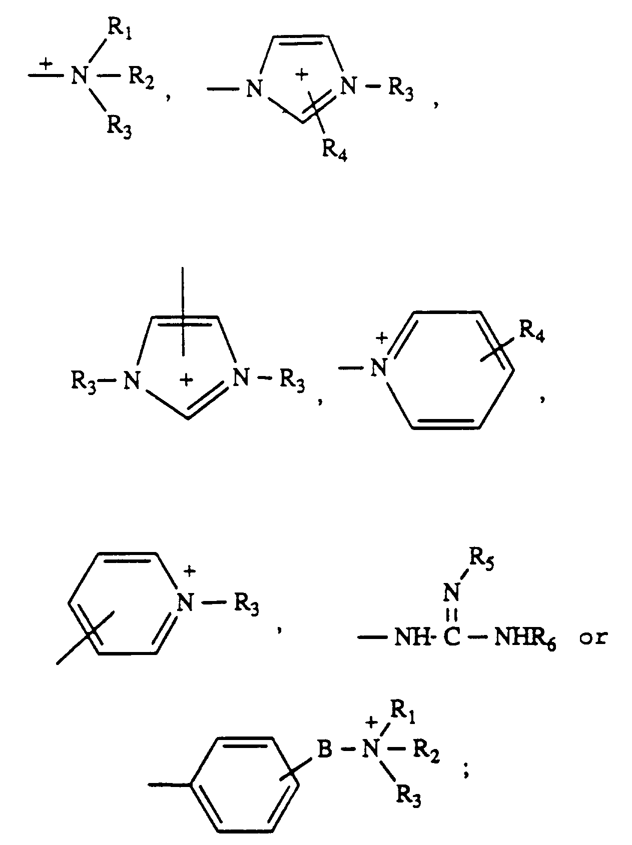
R is hydrogen, C₁-C₄ straight or branched alkyl, phenyl or when taken together with R₁ forms a saturated monoheterocyclic ring of from 5 to 6 members;
R₁, R₂ are the same or independently C₁-C₁₀ straight or branched chain alkyl which may be substituted by up to 3 substituents selected from 1 to 2 hydroxyl groups and one 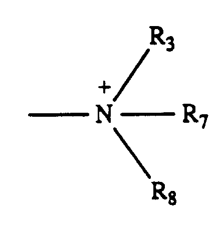
group, or when taken together form a saturated heterocyclic ring of from 5 to 6 members which may contain a 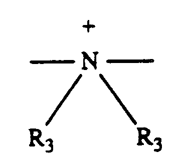
or -O- linkage;
R₃ is methyl, ethyl, benzyl which may be substituted by up to 3 substituents selected from hydroxyl, methoxy, halogen and C₁-C₆straight or branched chain alkyl, 1-naphthyl or 2-naphthyl;
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which may be substituted by one hydroxyl group or when taken together with an -O- linkage form the morpholine moiety;
- B: is a direct link or a C₁-C₄ straight or branched chain alkyl;
- n: is an integer in the range from about 50 to 500; and
- X⁻: is an anion forming a pharmaceutically acceptable salt.

The foregoing novel compounds of Formula I are inclusive of the provisos that
when R is hydrogen and Q is 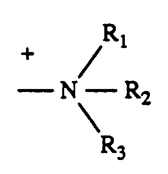
then R₃ cannot be methyl or ethyl when
a) one or both of R₁ and R₂ is a C₁-C₁₀ straight or branched chain unsubstituted alkyl,
b) R₁ and R₂ together form a 6 membered monoheterocyclic or dihetero cyclic oxygen containing ring.

It is to be understood that the definition of the compounds of Formulae I and II encompass all possible stereoisomers and mixtures thereof which possess the activity discussed below. In particular, it encompasses racemic modifications and any optical isomers which possess the individual activity.

As contemplated herein, the pharmaceutically acceptable anion X⁻ is inclusive of, for instance, halide (preferably chloride), acetate, citrate, propionate, phosphate and sulfate.

In the foregoing Formulae I & II, the term straight or branched alkyl group represents for instance methyl, ethyl, propyl, isopropyl, butyl, sec.-butyl and tertiary butyl.

As depicted in the following Schemes and in the Process Aspect and in the Formulae I and II the -C₂H₃- group can represent either the -CH-CH₂- group or the 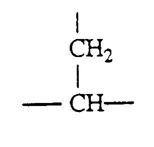
group. Thus in Formula II the groups can either be depicted as 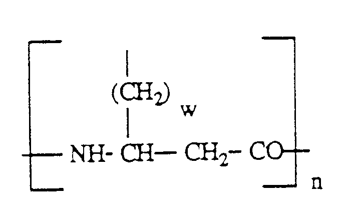
or be depicted as 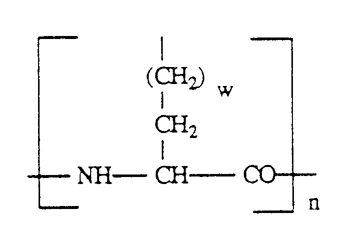
or even mixtures thereof.

The compounds of the invention have backbones derived from polyanhydroaspartic acid or polyglutamate. Considered as equivalents to this invention are co-polymers of the type as exemplified in Formula 7. Also considered as equivalents are those potentially branched or cross-linked polymers of the type as exemplified by Examples XII, XIII, and XVIII. In general, this invention includes homo- and copolymers having monomeric units of the formula [NH-C₂H₃A-CO] where A is (CH₂)_{w}-CO-HN-(CH₂)_{y} -(CH)ₜR-(CH₂)_{z}-Q, wherein the variables are as defined above, and wherein individual monomeric units may be the same or different and the overall number of monomeric units is as defined above.

The compounds which follow are some of those which serve to exemplify various aspects of the invention described herein.
A. Poly[imino[1-[2-[[[5-[(3-chlorophenyl)methyl-2-hydroxyethyl(propyl)ammonio]-3-phenylpentyl]amino]carbonyl]-ethyl]-2-oxo-1,2-ethanediyl chloride]].
B. Poly[imino[1-[[[[5-[[2-[4-methylmorpholinium-4-yl]-ethyl]-2-hydroxyethyl(methyl)ammonio]pentyl]amino]carbonyl]methyl]-2-oxo-1,2-ethanediyl dicitrate]].
C. Poly[imino[1-[[[[[1-methyl-1-naphthalenylpiperidinium-4-yl]methyl]amino]carbonyl]methyl]-2-oxo-1,2-ethanediyl chloride]].
D. Poly[imino[1-[[[[[1-(phenylmethyl-1-[2-[4-methylmorpholinum-4-yl]ethyl]piperidinium-4-yl]methyl]amino]carbonyl]-methyl]-2-oxo-1,2-ethanediyl dichloride]].
E. Poly[imino[1-[[[[3-[[3-[ethyl(methyl)propylammonio]propyl]-2-(trimethylammonio)ethyl(methyl)ammonio]propyl]amino]carbonyl]methyl]-2-oxo-1,2-ethanediyl trichloride]].
F. Poly[imino[1-[2-[[[4-(1-ethyl-4,4-di(phenylmethyl)piperazinium-1-yl]butyl]amino]carbonyl]ethyl]-2-oxo-1,2-ethanediyl diphosphate]].
G. Poly[imino[1-[[[[3-[[[[(1-methylethylamino)1-methylethylimino]methyl]amino]methyl]pentyl]amino]carbonyl]-methyl]-2-oxo-1,2-ethanediyl]]hydrochloride.

### PROCESS ASPECT

The compounds of this invention can be prepared, in general, by standard techniques analogous to those known in the art. The general process is characterized in that polyanhydroaspartic acid of the Formula **1**, in which n is an integer from 15 to 500, is reacted with at least one amine of the Formula **2** where A is a non-quaternized Q lacking R₃. 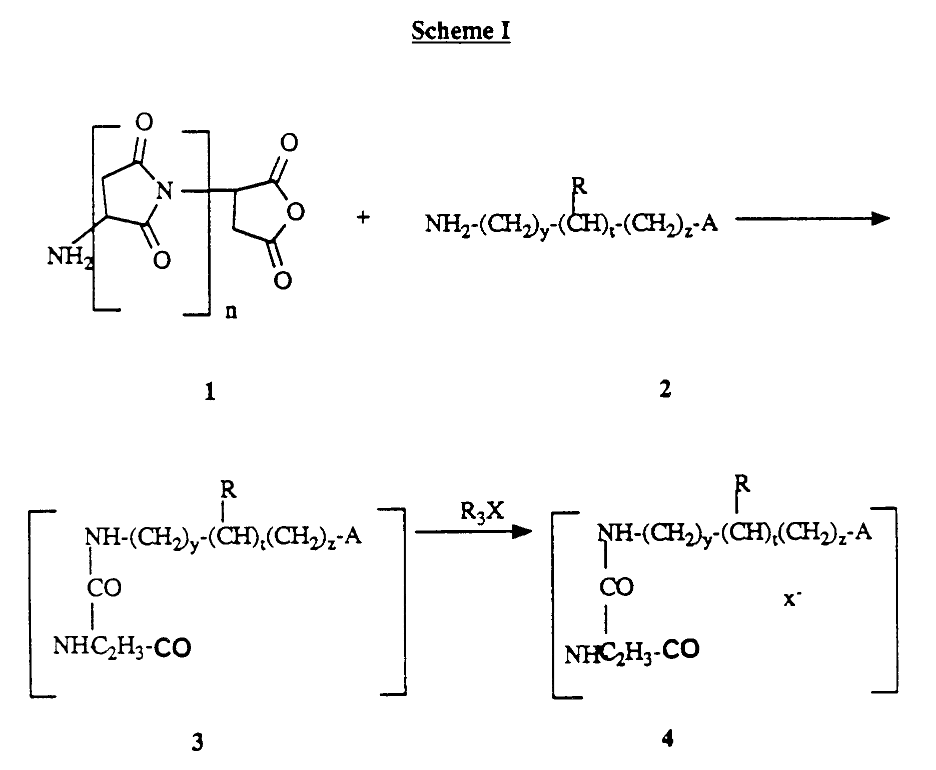
The amidation reaction can bring an amine onto all the reactive imide sites of the polyanhydroaspartic acid, or onto a fraction of said reactive sites, in which case the reaction can be followed by an amidation reaction with one or several amines having the Formula 2. For complete amidation reaction, m₁ moles of amine are being reacted per mole (monomer basis) of polyanhydroaspartic acid, m₁ being an integral or non-integral number between 1 and 15. In the case where more than one amine is reacted with polyanhydroaspartic acid, the amines can be added sequentially at a quantity of m₁ being a fraction between 0 and 1 for the first amine, and m₂ being equal to or greater than 1-m₁ for the second amine.

The amidation reactions are generally carried out in a solvent such as dimethylformamide (DMF), although dimethylsulfoxide (DMSO) can also be used. Reactions may be run at room temperature to about 75°C. Reactions times are generally in the range of 6-72 hours. The products of the amidation reaction may be isolated by, for example, precipitation from the reaction mixture with a non-solvent or by dilution with an aqueous acid, dialysis to remove low molecular weight products, and lyophilization. The second procedure provides salts of the intermediate polymeric amines. Alternatively, the reaction mixture can be used directly in the subsequent quaternization [or guanidine formation] reaction.

The polyanhydroaspartic acid can be obtained in accordance with the methods described in the literature. As for instance, French Patent 70.24831, the polyanhydroaspartic acid is prepared by heating aspartic acid in the presence of phosphoric acid under vacuum at 170°-200°C assuring a constant renewal of the reaction mass surface. This preparation can be carried out using a rotary evaporator to renew the surface.

The products from Scheme I of Formula **3** can be converted to the quaternary ammonium derivatives **4** by reaction with a suitable alkylating agent. The products of Formula **4** are most commonly obtained by quaternization of **3** with an excess of dimethyl sulfate, methyl chloride, methyl iodide or other alkylating agent (e.g. benzyl bromide) in, for example, DMF or methanol at room temperature. An insoluble base such as potassium or calcium carbonate may be added as a proton scavanger. This is necessary if the group A is -NH₂ or NHR, and also if a salt form of Formula 3 (such as hydrochloride) is used in the reaction. Such bases may be used in excess, from 1 to 5 equivalents. In the cases where the quaternization is difficult, heating the reaction mixture up to 75°C in DMF, or using a more reactive alkylating agent such as methyltrifluoromethanesulfonate may be appropriate.

Insofar as the anion X⁻ is concerned in the foregoing scheme, used in conjunction with describing the polymer **4**, a wide variety of anions are used herein, a criterion being the pharmaceutically acceptable nature of such. Suitable anions include, for example, halides, acetate, citrate, propionate, sulfate and phosphate, chloride being preferred.

Replacement of the counter anion X⁻ in these polymers with other pharmaceutically acceptable anions may be accomplished by methods well known to the art. For example, diluted polymer solutions can be treated with insoluble anion exchange resins in the hydroxide form. The hydroxide salt of the polymer can then be neutralized with the appropriate acid Preferably, diluted polymer solutions can be directly converted to the chloride anion form by treating two or more times with an excess of aqueous hydrochloric acid followed by dialysis.

The aqueous polymer solutions can be dialyzed to ensure no low molecular weight components remain. The dialysis can be performed, for example, with a Millipore Minitan Ultrafiltration System using a polysulfone membrane with a retentive molecular weight cutoff of approximately 10,000 NMWL. The aqueous solution can then be lyophilized to afford the polymers as amorphous solids.

This process can be monitored by using analysis of ionic halogen and relative ratios of the other elemental constituents, titration of residual tertiary amine and NMR determination. Relative molecular weights can be determined by gel permeation chromatography. 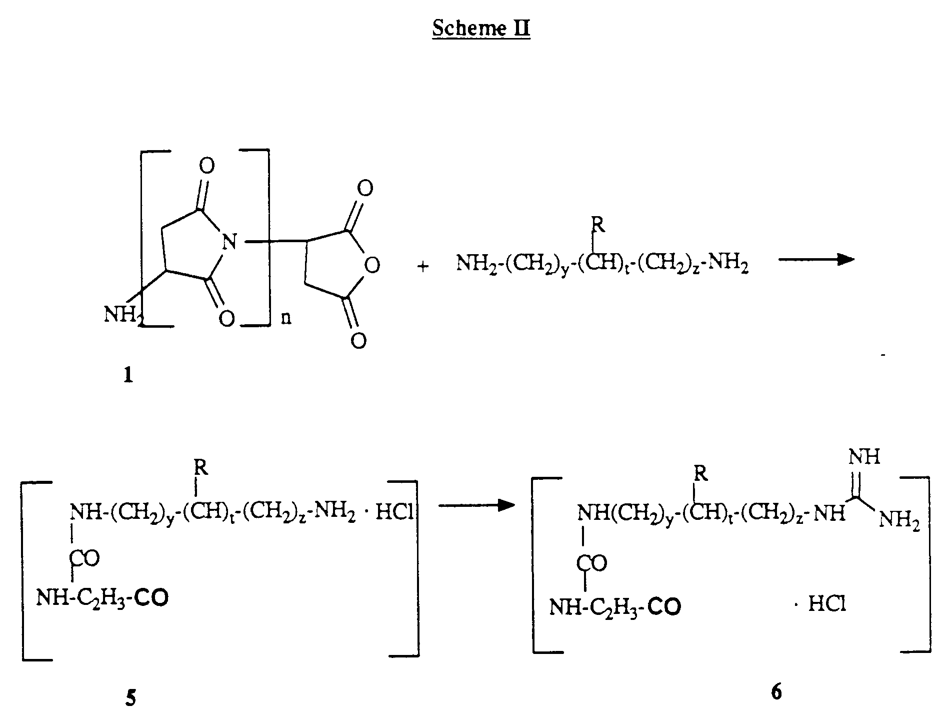
In the foregoing Scheme II where Q is a guanidinium functionality (Formula **6**), the compounds of this type may be prepared by reaction of an excess of a primary diamine with polyanhydroaspartic acid. The intermediate of Formula **5** may be isolated as the hydrochloride salt and converted to the corresponding guanidinium derivative by reaction with a guanidinating agent known to the art, such as S-methylisothiouronium iodide in the presence of base.

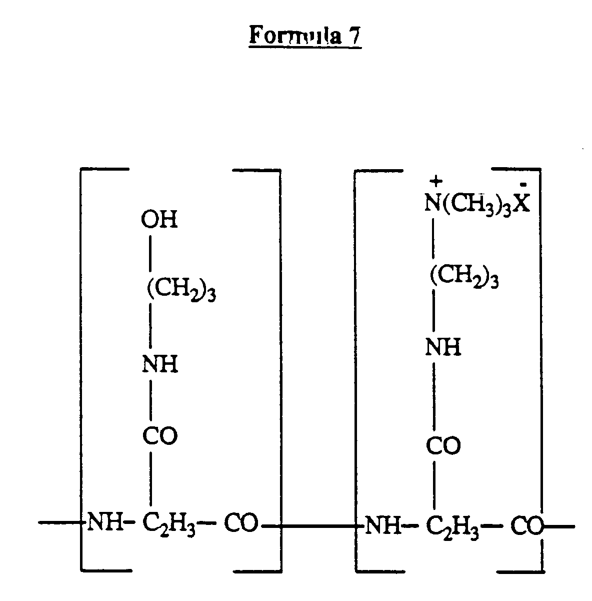

Copolymers can be developed when polyanhydroaspartic acid is reacted with two or more different amines of the Formula **2**. Copolymers of the foregoing Formula **7** may also be similarly developed by first reacting with a quantity m₁ of 3-aminopropanol, m₁ being a fraction between 0 and 1. The intermediate may then be reacted with a quantity m₂ of 3-dimethylaminopropylamine, m₂ being equal to or greater than 1-m₁. The copolymer can then be quaternized by the previously described method.

Copolymers that are potentially branched or cross-linked may be prepared by first a reaction of polyanhydroaspartic acid with a quantity m₁ of a primary diamine, m₁ being a fraction between 0 and 1 equivalents. The intermediate can then be reacted with a quantity m₂ of a second amine of Formula **2**, m₂ being equal to or greater than 1-m₁ equivalents for the second amine. These potentially branched or cross-linked copolymers can then be quaternized by the previously described method. Alternatively, polymers of Formula **3** can be reacted with various amounts of cross-linking reagents, for example, 1,4-dichloro-2-butene, and a quaternizing agent, to afford co-polymers that are potentially branched or cross-linked.

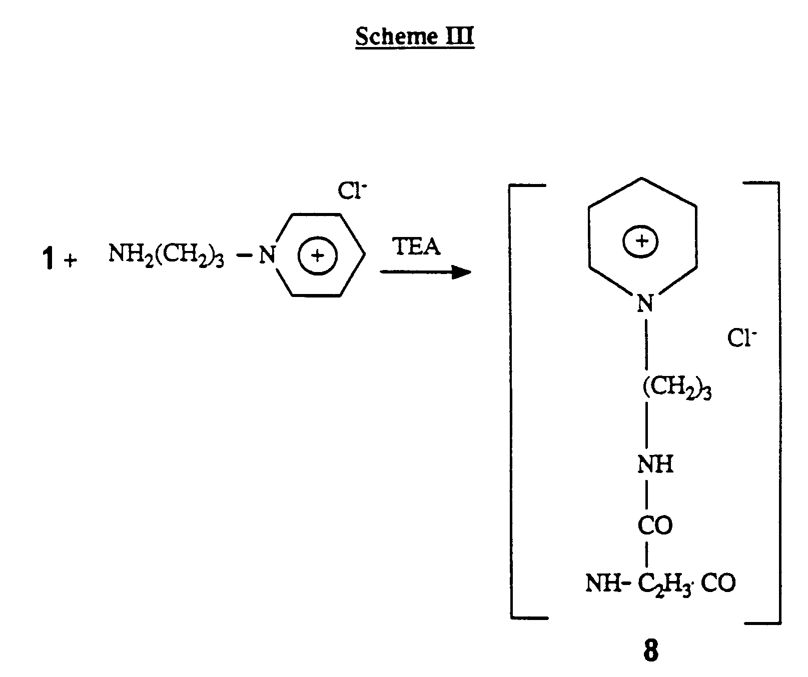

In such instances where Q is an N-substituted pyridinium derivative (Formula 8), the polymeric quaternized pyridinium derivative may be prepared in one step as in the foregoing Scheme III, by reaction of 3-aminopropylpyridinium chloride hydrochloride and triethylamine with polyanhydroaspartic acid.

In such instances where Q is a diquaternary derivative (Formula 9), the polymeric diquaternized derivatives may be prepared by the generalized process of amidation with excess amine (triamine) followed by quaternization with excess alkylating agent as exemplified in the following Scheme IV.

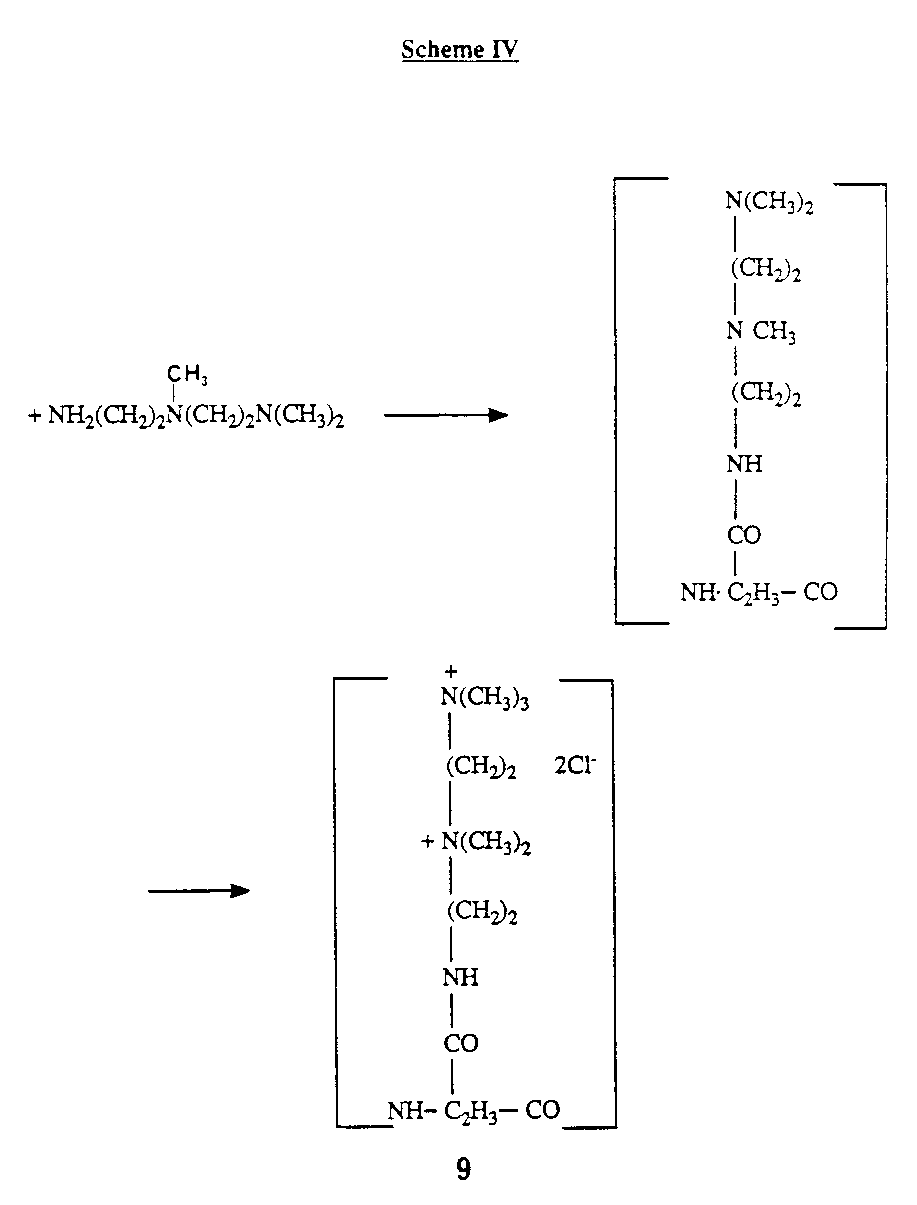

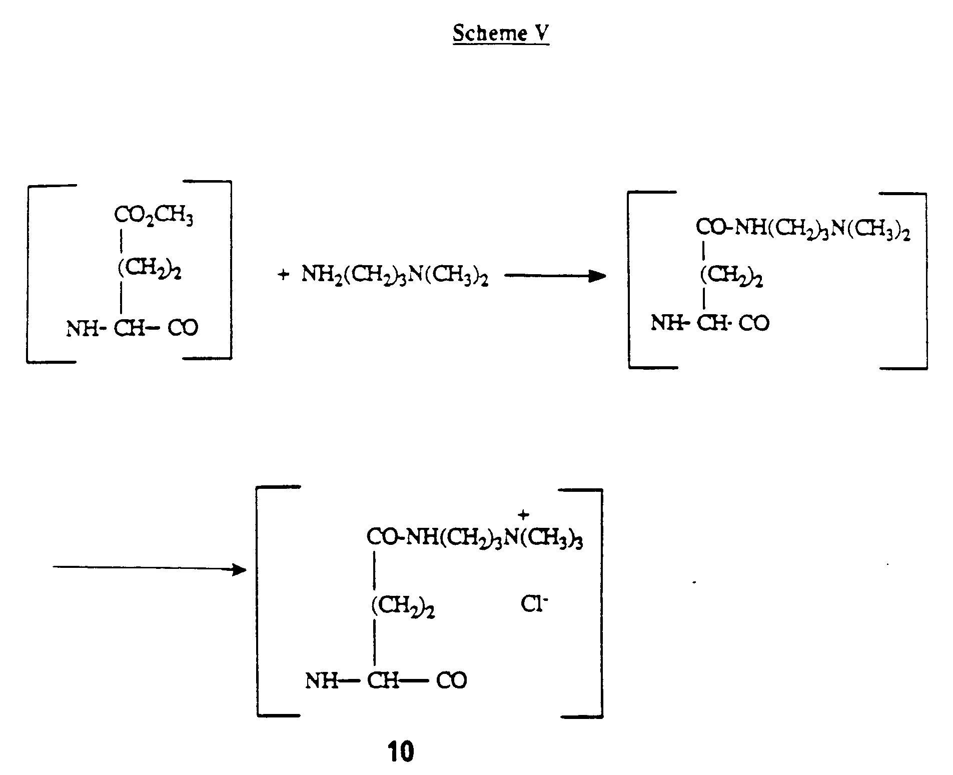

In the above Scheme V, polyglutamide derivatives (Formula 10) may be prepared by standard techniques analogous to those known in the art. Poly(γ-methyl L-glutamate) can be reacted with N,N-dimethyl-1,3-propanediamine, quaternized with dimethyl sulfate, and ion exchanged with hydrochloric acid to afford a polymer of Formula 10.

### Method of Use and Pharmaceutical Composition Aspect

Clinical results demonstrating that in man a 1% reduction in serum cholesterol level results in a 2% reduction in coronary heart disease incidence have led the National Cholesterol Education Program Expert Panel to designate bile acid sequestrants as preferred first line therapy in familial hyperlipidemia patients who are at high risk of coronary heart disease, even ahead of systemic lipid lowering drugs such as HMG-CoA-reductase inhibitors (lovastatin). However, the observed clinical synergy between bile acid sequestrants and HMG-CoA-reductase inhibitors in the reduction of serum cholesterol suggests a further importance for the future of bile acid sequestrant therapy.

The liver is the primary source of the plasma cholesterol produced in humans. Bile acids are major end products of cholesterol catabolism which aid in fat digestion. A bile acid pool is maintained in the body (by enterohepatic cycling), a portion of which is normally excreted via the feces daily. Bile acid sequestrants act by definition to facilitate the removal of bile acids from their normal enterohepatic cycle in the G.I tract. This modification of bile acid excretion has the effect in certain mammals including man of enhancing the conversion of cholesterol to bile acids; their removal increases the rate of hepatic synthesis and metabolism of cholesterol. In addition, however, an increase in liver β-lipoprotein receptors occurs as well as a compensatory increase in the rate of hepatic biosynthesis of cholesterol both of which serve to maintain hepatic cholesterol balance. More importantly a resultant lowering of serum low density lipoproteins and cholesterol is observed. In other words, the lower level of available bile acids (from sequestration) in turn, requires the biosynthesis of replacement bile acids from cholesterol. It is estimated that about one third of cholesterol turnover in the body is attributable to bile acid synthesis. Forcing the body to make more bile acids is therefore a means of using up plasma and tissue stores of cholesterol.

The two approved bile acid sequestrants on the market are cholestyramine and colestipol. The main problems with these two agents, which are resins bearing quaternary ammonium groups, is their sandy, gritty consistency (they are water insoluble), their large daily dosing (4-32 grams) and fish like-odor in the case of cholestyramine. Their major side-effects are bloating, flatulence and constipation which makes 100% patient compliance in terms of the necessary chronic therapy nearly impossible to achieve.

The object of the compounds of this invention is to provide hypolipidemic agents which will be easy to administer thereby allowing for greater patient compliance to achieve the full efficacy potential of these agents. As hypolipidemic agents they will reduce the lipids in a hyperlipidemic mammal especially humans in need of such lipid lowering. These compounds are non-systemic, water soluble polymers which act as lipid lowering agents by not only acting as bile acid sequestrants thereby lowering serum cholesterol, but they have also been found to lower triglycerides.

The compounds of this invention were tested for efficacy in several models. The following three in vivo models illustrate some of the lipid effects of illustrative compounds.
I. Rat Cholesterol Synthesis Model: Rats are fed 7 days ad libitum on chow supplemented with 10% lard, 0.75% methionine (control) and test substance at X% of feed. A dose of ¹⁴C-acetate is administered in and the animals are sacrificed 50 minutes later, bled, and plasma is analyzed for ¹⁴C-cholesterol after lipid saponification. Increase in ¹⁴C-cholesterol synthesis is measured as the response to bile acid (BA) excretion promoted by bile acid sequestrants (BAS). Potency relative to a known BAS compound, cholestyramine, (Ch), is measured from the dependence of plasma ¹⁴C-cholesterol increase on percent of drug in feed.
The compounds tested and for which results are given in the following Table I are as follows:
Poly[imino[1-[[[[3-(trimethylammonio)propyl]amino]carbonyl]methyl]-2-oxo-1,2-ethanediyl chloride]] (Compound A);
Poly[imino[1-[[[[2-[[2-(trimethylammonio)ethyl]dimethylammonio]ethyl]amino]carbonyl]methyl]-2-oxo-1,2-ethanediyl chloride]] (Compound B);
Poly[imino[1-[[[[2-[[3-(trimethylammonio)propyl]dimethylammonio]ethyl]amino]carbonyl]methyl]-2-oxo-1,2-ethanediyl chloride]] (Compound C); and
Poly[imino[1-[[[[2-[bis(2-hydroxyethyl)methylammonio]ethyl]amino]carbonyl]methyl]-2-oxo-1,2-ethanediyl chloride]] (Compound D).

**TABLE I**

| **Cholesterol synthesis model** | | | | |
|---|---|---|---|---|
| Compound | Dose (% Diet) | In Vitro Bile Acid Binding Capacity meq/g | % Charge From Control | Ratio of Activity Relative To Cholestyramine |
| Cholestyramine | 1% | 4.1 | 77 | 1.00 |
| Compound A | 1% | 3.7 | 91 | 1.18 |
| Compound A | 2% | 3.7 | 358 | 1.00 |
| Compound B | 1% | 5.83 | 88 | 1.14 |
| Compound C | 2% | 4.85 | 279 | 0.78 |
| Compound D | 2% | 2.95 | 187 | 0.52 |

II. Hyperlipidemic Hamster Model: A similar feeding protocol to that used for rats uses chow supplemented with 10% lard and 0.0625% cholesterol with and without test drug. After 7 days of feeding, animals are sacrificed and plasma total cholesterol, HDL cholesterol and triglycerides are measured. Bile is collected from the gall bladder for HPLC analysis of BA composition. ED₃₀ for reduction of plasma cholesterol is determined from the fall in cholesterol from 250-300 to 100-125 mg/dg (the homeostasis level). Cholestyramine and colestipol were employed as standards.
The compounds tested for which results are given in the following table II are as follows:
Compound A,
Compound B; and
Poly[imino[1-[[[[3-[dimethyl(phenylmethyl)ammonio]propyl]aminocarbonyl]methyl]-2-oxo-1,2-ethanediyl chloride]] Compound E.

**Table II**

| **The Effect of Compounds on Plasma Cholesterol in the Male Hamster Fed Saturated Triglycerides and Cholesterol** | |
|---|---|
| Drug | Ratio of Potency in reductions of plasma total cholesterol Cholestyramine = 1 |
| Colestipol | 0.9 |
| (Compound A) | 2.35 |
| (Compound B) | 1.12 |
| (Compound E) | 0.82 |

III. Normal Lipemic Hamster Model: Male hamsters are fed for a period of three weeks ad libitum on rodent chow that is not supplemented with additional lipids and that contained the test substances at 1% by weight of feed. At the end of the feeding period the animals were sacrificed and plasma total cholesterol, HDL cholesterol and triglycerides are measured. Bile is collected from the gall bladder for HPLC analysis of BA composition. Livers are perfused with fluoride containing buffer and microsomes are prepared from them for the analysis of the activities of the enzymes 3-hydroxy-3-methylglutaryl CoA reductase and cholesterol 7-alpha hydroxylase. The observations for the animals treated with test compound are compared to the results from a control group that was not fed drug. Cholestyramine was used in the study as a known BAS agent.

**Table III**

| Reduction of Plasma Lipids in Male Hamsters Fed a Rodent Chow Diet Without Additional Lipids | | | |
|---|---|---|---|
| Test/Group No. | Plasma | | |
| | Cholesterol mg/dL | HDL Cholesterol mg/dL | Triglyceride mg/mL |
| Current Control | 190.3 | 84.8 | 149.1 |
| Cholestyramine | 123.6 | 77.2 | 147.0 |
| Compound A | 149.7 | 78.5 | 112.0 |
| Compound B | 154.6 | 74.1 | 106.0 |
| Compound E | 154.7 | 74.2 | 85.0 |

It is contemplated that the compounds of this invention - for example Compound A, B, C, D and E as illustrated above can be administered orally to mammals most especially humans to act not only as sequestrants for bile acids but as triglyceride lowering agents. That such oral administration will be in an amount effective to debase the amount of lipids in the mammal in need of such reduction. In addition to the foregoing, the compounds of the present invention have been found to be effective in depleting bile and fecal deoxycholate with a concomitant increase in chenodeoxycholate. This is in contradistinction to the action of cholestyramine which reduces chenodeoxycholate. This dual effect of depletion of deoxycholate and concomitant increase of chenodeoxycholate results in a reduction of the lithogenicity of bile. Thus the polymers of this invention are useful in dissolving, delaying progression and/or blocking the formation of gallstones in humans. The compounds of the present invention show potential for blocking synthesis of atherogenic lipoproteins, e.g., VLDL and for broader utility in the treatment of hyperlipidemias and atherosclerosis in humans. The compounds when administered orally would be cojoined with pharmaceutically acceptable carriers when necessary. It is contemplated that such carriers might for example involve flavoring agents especially if the preparation is in the form of a syrup. They might also be given as a capsule or after lyophilization compressed into tablets, or packaged as individual sachets to be admixed with some liquid (e.g. fruit juice) or packed in bulk form. It is also contemplated that the dosing would be in the range of 5-35 gm per day.

The invention described herein above is illustrated below in the Examples, which, however are not to be construed as limiting the scope of this invention.

### EXAMPLE I

### Poly[imino[1-[[[[3-(trimethylammonio)propyl]amino]carbonyl]methyl]-2-oxo-1,2-ethanediyl chloride]]

3.5g of polyanhydroaspartic acid is dissolved in 30 mL of anhydrous DMF and added dropwise to 9.2g of N,N-dimethyl- 1,3-propanediamine while keeping the temperature of the reaction mixture below 30°C. The mixture is stirred under a nitrogen atmosphere for 18 hours at room temperature, then precipitated with a mixture of ether and petroleum ether. The precipitate is dissolved in a minimum amount of methanol and reprecipitated with ether. The precipitate is dissolved in 100 mL of methanol, and to this solution is added 25.0g of potassium carbonate and 22.7g of dimethyl sulfate over 0.5 h. The mixture is stirred 18 h at room temperature, then filtered and precipitated with ether. The precipitate is dissolved in 500 mL H₂O and acidified with 30 mL concentrated hydrochloric acid. The solution is dialyzed while maintaining the volume constant by addition of deionized water until the effluent is pH=3. An additional 30 mL of concentrated hydrochloric acid is added and the solution is redialyzed until the effluent is pH=6. The solution is concentrated by dialysis to 100 mL and lyophilized to afford the water soluble title compound.

| | | |
|---|---|---|
| % Cl | Calc. 14.20 | Found 13.19. |
| Cl/N | Calc. 0.84 | Found 0.84 |
| C/N | Calc. 2.86 | Found 2.89 |

### Example II

In a manner similar to Example I, the following compounds
a) N,N-dimethylethanediamine,
b) N,N-dimethyl-1,4-benzenedimethanamine,
c) 1-pyrrolidinethanamine,
d) 4-morpholinepropanamine,
e) 1-piperidinethanamine,
f) N,N-dibutylpropanediamine,
g) N,N-diethylpropanediamine,
h) N,N-diethylhexanediamine,
i) N,N-dimethylbutanediamine,
j) [4-(diethylamino)phenyl]methanamine,
k) N,N-bis(2-hydroxyethyl)ethanediamine and
l) 1-1H-imidazole propanamine,
are reacted with polyanhydroaspartic acid to produce respectively:
m) poly[imino[1-[[[[2-(trimethylammonio)ethyl]amino]carbonyl]methyl]-2-oxo-1,2-ethanediyl chloride]],
n) poly[imino[1-[[[[4-[(trimethylammonio)methyl]phenyl]methyl]amino]carbonyl]methyl]-2-oxo-1,2-ethandiyl chloride]],
o) poly[imino[1-[[[[2-[1-methylpyrrolidinium-1-yl]ethyl]amino]carbonyl]methyl]-2-oxo-1,2-ethanediyl chloride]],
p) poly[imino[1-[[[[3-[4-methylmorpholinium-4-yl]propyl]-amino]carbonyl]methyl]-2-oxo-1,2-ethanediyl chloride]],
q) poly[imino[1-[[[[2-[1-methylpiperidinium-1-yl]ethyl]amino]carbonyl]methyl]-2-oxo-1,2-ethanediyl chloride]],
r) poly[imino[1-[[[[3-[dibutyl(methyl)ammonio]propyl]amino]carbonyl]methyl]-2-oxo-1,2-ethanediyl chloride]],
s) poly[imino[1-[[[[3-[diethyl(methyl)ammonio]propyl]amino]carbonyl]methyl]-2-oxo-1,2-ethanediyl chloride]],
t) poly[imino[1-[[[[6-(trimethylammonio)hexyl]amino]carbonyl]methyl]-2-oxo-1,2-ethanediyl chloride]],
u) poly[imino[1-[[[[4-(trimethylammonio)butyl]amino]carbonyl]methyl]-2-oxo-1,2-ethanediyl chloride]],
v) poly[imino[1-[[[[4-(trimethylammonio)phenyl]methyl]amino]carbonyl]methyl]-2-oxo-1,2-ethanediyl chloride]],
w) poly[imino[1-[[[[2-[bis(2-hydroxyethyl)methylammonio]ethyl]amino]carbonyl]methyl]-2-oxo-1,2-ethanediyl chloride]] and
x) poly[imino[1-[[[[3-(3-methylimidazolium-1-yl)propyl]amino]carbonyl]methyl]-2-oxo-1,2-ethanediyl chloride]].

### Example III

### Poly[imino[1-[[[[3-[(dimethyl)phenylmethylammonio]-propyl]aminocarbonyl]methyl]-2-oxo-1,2-ethanediyl chloride]]

3.5g of polyanhydroaspartic acid is dissolved in 30 mL of anhydrous DMF and added dropwise to 9.2g of N,N-dimethyl-1,3-propanediamine while keeping the temperature of the reaction mixture below 30°C. The mixture is stirred under a nitrogen atmosphere for 18 h at room temperature, then precipitated with a mixture of ether and petroleum ether. The precipitate is dissolved in a minimum amount of methanol and reprecipitated with ether. The precipitate is dissolved in 200 mL of methanol, and to this solution is added 25.0g of potassium carbonate and 22.8g of benzyl chloride. The mixture is stirred for 48 h at 50°C, then is filtered and precipitated with ether. The precipitate is dissolved in 500 mL H₂O and acidified with 30 mL concentrated hydrochloric acid. The solution is dialyzed while maintaining the volume constant by addition of deionized water until the effluent is pH=6. The solution is concentrated by dialysis to 100 mL and lyophilized to afford the water soluble title compound.

| | | |
|---|---|---|
| % Cl | Calc., 10.88 | Found 8.99 |
| Cl/N | Calc., 0.84 | Found 0.78 |
| C/N | Calc., 4.57 | Found 4.61 |

### Example IV

### Poly[imino[1-[[[[2-[(dimethyl)phenylmethylammonio]ethyl]amino]carbonyl]methyl]-2-oxo-1,2-ethanediyl chloride]]

In a manner similar to Example III, 2-(dimethylamino)ethylamine is reacted with polyanhydroaspartic acid and then with benzyl chloride to produce the water soluble title compound.

### Example V

### Poly[imino[1-[[[[2-(1-methylpyridinium-2-yl)ethyl]amino]carbonyl]methyl]-2-oxo-1,2-ethanediyl chloride]]

1.5g of polyanhydroaspartic acid is dissolved in 25 mL of anhydrous DMF and is added to 4.7g of 2-pyridinethanamine. The mixture is stirred under a nitrogen atmosphere at 70°C for 18 h, then precipitated with ether. The precipitate is dissolved in a minimum amount of methanol and reprecipitated with ether. The precipitate is dissolved in 35 mL of DMF, and to this solution is added 6.0g of methyl trifluoromethanesulfonate. The mixture is stirred 18 h at room temperature, then precipitated with ether. The precipitate is dissolved in 300 mL H₂O and acidified with 10 mL concentrated hydrochloric acid. The solution is dialyzed as per Ex.I until the effluent is pH=3. An additional 10 mL of concentrated hydrochloric acid is added and the solution is dialyzed until the effluent is pH=6. The solution is concentrated by dialysis to 100 mL and lyophilized to afford the water soluble title compound.

| | | |
|---|---|---|
| % Cl | Calc., 13.14 | Found, 9.13. |
| Cl/N | Calc., 0.84 | Found, 0.60 |
| C/N | Calc., 3.43 | Found, 3.36 |

### Example VI

### Poly[imino[1-[[[[3-(1,3-dimethylimidazolium-2-yl)propyl]amino]carbonyl]methyl]-2-oxo-1,2-ethanediyl chloride]]

1.5g of polyanhydroaspartic acid is dissolved in 35 mL of anhydrous DMF and added to 5.4g of 1-methyl-2-1H-imidazolepropanamine. The mixture is stirred under a nitrogen atmosphere at 75°C for 40 h, then precipitated with a mixture of ether and petroleum ether. The precipitate is dissolved in a minimum amount of methanol and reprecipitated with ether. The precipitate is dissolved in 200 mL of methanol, and to this solution is added 10.6g of potassium carbonate and 9.7g of dimethyl sulfate. The mixture is stirred 10 days at room temperature then 3 days at 50°C. The reaction mixture is filtered and precipitated with ether. The precipitate is dissolved in 300 mL H₂O and acidified with 10 mL concentrated hydrochloric acid. The solution is dialyzed as per Ex.I until the pH=3. An additional 10 mL of concentrated hydrochloric acid is added and the solution is dialyzed until the effluent is pH=6. The solution is concentrated via dialysis to 100 mL and lyophilized to afford the water soluble title compound.

| | | |
|---|---|---|
| % Cl | Calc., 12.36 | Found, 8.53. |
| Cl/N | Calc., 0.63 | Found, 0.47 |
| C/N | Calc., 2.57 | Found, 2.63 |

### Example VII

### Poly[imino[1-[[[[2-(1,3-dimethylimidazolium-4-yl)ethyl]amino]carbonyl]methyl]-2-oxo-1,2-ethanediyl chloride]]

3.1g of polyanhydroaspartic acid is dissolved in 35 mL of anhydrous DMF and added to 8.9g of 4-1H-imidazolethanamine. The mixture is stirred under a nitrogen atmosphere for 24 h at 75°C, cooled to room temperature, then precipitated with ether. The precipitate is dissolved in a minimum amount of methanol and reprecipitated with ether. The precipitate is dissolved in 500 mL of DMF, and to this solution is added 130g of potassium carbonate followed by 120g of dimethyl sulfate dropwise over 2 h. The mixture is stirred for 18 h at 75°C, then is filtered and precipitated with ether. The precipitate is dissolved in 150 mL 1N hydrochloric acid, and dialyzed as per Ex.I until the effluent is pH=3. An additional 150 mL of 1N hydrochloric acid is added and the solution is redialyzed until the effluent is pH=6. The solution is concentrated by dialysis to 100 mL and lyophilized to afford the water soluble title compound.

| | | |
|---|---|---|
| % Cl | Calc., 13.10 | Found, 9.43. |
| Cl/N | Calc., 0.63 | Found, 0.49 |
| C/N | Calc., 2.36 | Found, 2.45 |

### Example VIII

### Poly[imino[1-[[[[3-[(aminoiminomethyl)amino]propyl]amino]carbonyl]methyl]-2-oxo-1,2-ethanediyl hydrochloride]]

35g of polyanhydroaspartic acid is dissolved in 300 mL of anhydrous DMF and added to a solution of 266g 1,3-propanediamine in 500 mL of DMF. The mixture is stirred under a nitrogen atmosphere for three days at room temperature, then precipitated with ether. The precipitate is dissolved in 6 L of water, acidified to pH=1, dialyzed until the effluent is pH=3, then lyophilized to afford 62.3g of a water soluble solid 98.4g of methylisothiouronium iodide is dissolved in 480 mL of 1.0 M sodium hydroxide and 750 mL of methanol. The above water soluble solid is added, and the mixture is stirred for five days under a nitrogen atmosphere at room temperature. The reaction mixture is then acidified with 625 mL of 6N hydrochloric acid, diluted to 10 L with water, and then dialyzed as per Ex.I until the effluent is pH=3. An additional 625 mL of 6N hydrochloric acid is added and the solution is redialyzed until the effluent is pH=6. The solution is concentrated by dialysis to 2L and lyophilized to afford the water soluble title compound

| | | |
|---|---|---|
| % Cl | Calc., 14.20 | Found, 11.11. |
| Cl/N | Calc., 0.51 | Found, 0.43 |
| C/N | Calc., 1.37 | Found, 1.47 |

### Example IX

In a manner similar to Example VIII, the following reactants:
a) 1,2-ethanediamine
b) 1,4-butanediamine
are reacted with polyanhydroaspartic acid and then with methyl isothiouronium chloride (or iodide) and bases to produce respectively:
c) poly[imino[1-[[[[2-[(aminoiminomethyl)amino]ethyl]amino]carbonyl]methyl]-2-oxo-1,2-ethanediyl hydrochloride]] and
d) poly[imino[1-[[[[4-[(aminoiminomethyl)amino]butyl]amino]carbonyl]methyl]-2-oxo-1,2-ethanediyl hydrochloride]].

### Example X

### Poly[imino[1-[[[[2-[[2-(trimethylammonio)ethyl]dimethylammonio]ethyl]amino]carbonyl]methyl]-2-oxo-1,2-ethandiyl-dichloride]]

4.0g of polyanhydroaspartic acid is dissolved in 40 mL of anhydrous DMF and added dropwise to 7.2g of N-(2-aminoethyl)-N,N',N'-trimethylethanediamine while keeping the temperature of the reaction mixture below 30°C. The mixture is stirred under a nitrogen atmosphere for 18 h. To this mixture is added 20.0g of potassium carbonate followed by 18.2g of dimethyl sulfate over 0.5 h. The mixture is then stirred 48 h at room temperature, filtered, and precipitated with ether. The precipitate is dissolved in 500 mL H₂O and acidified with 66 mL of concentrated hydrochloric acid The solution is dialyzed as per Ex.I until the effluent is pH=3. An additional 66 mL of concentrated hydrochloric acid is added and the solution is redialyzed until the effluent is pH=6. The solution is concentrated by dialysis to 100 mL and lyophilized to afford the water soluble title compound.

| | | |
|---|---|---|
| % Cl | Calc., 20.65 | Found, 15.37. |
| Cl/N | Calc., 1.27 | Found, 1.04 |
| C/N | Calc., 2.79 | Found, 3.05 |

### Example XI

In a manner similar to Example X, the following reactants:
a) N-(2-aminoethyl)-N-,N',N'-trimethyl-1,3-propanediamine and
b) 4-methyl-1-piperazinethanamine,
are reacted with polyanhydroaspartic acid and quaternized with dimethyl sulfate to produce respectively:
c) poly[imino[1-[[[[2-[[3-(trimethylammonio]propyl]dimethylammonio]ethyl]amino]carbonyl]methyl]-2-oxo-1,2-ethanediyl dichloride]] and
d) poly[imino[1-[[[[2-(1,4,4-trimethylpiperizinium-1-yl)ethyl]amino]carbonyl]methyl]-2-oxo-1,2-ethanediyl dichloride]].

### Example XII

### Poly[imino[1-[[[[3-(trimethylammonio)propyl]amino]carbonyl]methyl]-2-oxo-1,2-ethanediyl chloride]], [RS], Crosslinked with 15% 1,3-propanediamine

5.0g of polyanhydroaspartic acid is dissolved in 50 mL of DMSO. 0.57g of 1,3-propanediamine (A) is added and the mixture is heated for 2 h at 75°C under a nitrogen atmosphere. The reaction mixture is cooled to room temperature and 28.6g of N,N-dimethyl-1,3-propanediamine (B)is added. The mixture is stirred for 1 h at room temperature, 18 h at 75°C, then cooled to room temperature and precipitate with ether. The precipitate is dissolved in 50 mL of DMF, and to this solution is added 35.6g of potassium carbonate and 32.5g of dimethyl sulfate. The mixture is stirred for 1 h at 75°C, then 18 h at room temperature and then filtered and precipitated with ether. The precipitate is dissolved in 500 mL H₂O and acidified with 45 mL concentrated hydrochloric acid. The solution is dialyzed as per Ex.I until the effluent is pH=3. An additional 45 mL of concentrated hydrochloric acid is added and the solution is redialyzed until the effluent is pH=6. The solution is concentrated by dialysis to 100 mL and lyophilized to afford the water soluble title compound.

| | | |
|---|---|---|
| % Cl | Calc., 13.59 | Found, 10.68. |
| Cl/N | Calc., 0.77 | Found, 0.64 |
| C/N | Calc., 2.72 | Found, 2.82 |

### Example XIII

Polymers that may potentially have varying degrees of branching or cross-linking can be prepared by adjusting the ratio of the diamines. Thus polymers wherein the reacting diamine (A):(B) ratios are:
a) 1:99,
b) 2.5 : 97.5,
c) 5: 95,
d) 7.5 : 92.5 and
e) 10 : 90,
can be prepared in a manner similar to Example XII by reacting the respective amounts of 1,3-propanediamine and N,N-dimethylpropanediamine with polyanhydroaspartic acid to obtain respectively:
f) poly[imino[1-[[[[3-(trimethylammonio)propyl]amino]carbonyl]methyl]-2-oxo-1,2-ethanediyl chloride]], [RS], crosslinked with 1% 1,3-propanediamine,
g) poly[imino[1-[[[[3-(trimethylammonio)propyl]amino]carbonyl]methyl]-2-oxo-1,2-ethanediyl chloride]], [RS], crosslinked with 2.5% 1,3-propanediamine,
h) poly[imino[1-[[[[3-(trimethylammonio)-propyl]amino]carbonyl]methyl]-2-oxo-1,2-ethanediyl chloride]], [RS], crosslinked with 5% 1,3-propanediamine,
i) poly[imino[1-[[[[3-(trimethylammonio)-propyl]amino]carbonyl]methyl]-2-oxo-1,2-ethanediyl chloride]], [RS], crosslinked with 7.5% 1,3-propanediamine, and
j) poly[imino[1-[[[[3-(trimethylammonio)-propyl]amino]carbonyl]methyl]-2-oxo-1,2-ethanediyl chloride]], [RS], crosslinked with 10% 1,3-propanediamine.

### Example XIV

### Poly[imino[1-[[[[3-hydroxypropyl]amino]carbonyl]methyl]-2-oxo-1,2-ethanediyl]imino[1-[[[[3-(trimethylammonio)propyl]amino]carbonyl]methyl]-2-oxo-1,2-ethanediyl chloride]]

4.0g of polyanhydroaspartic acid is dissolved in 50 mL of anhydrous DMF and added to 1.55g of 3-aminopropanol (A). The mixture is stirred at room temperature for 3h, and 4.21g of N,N-dimethyl-1,3-propanediamine (B) is added and the reaction mixture is stirred for 18 h. To this solution is added 45.6g of potassium carbonate and 41.6g of dimethyl sulfate over 0.5 h. The mixture is stirred 18 h at room temperature, filtered, and precipitated with ether. The precipitate is dissolved in 500 mL H₂O and acidified with 30 mL of concentrated hydrochloric acid The solution is dialyzed as per Ex.I until the effluent is pH=3. An additional 30 mL of concentrated hydrochloric acid is added and the solution is redialyzed until the effluent is pH=6. The solution is concentrated by dialysis to 100 mL and lyophilized to afford a water soluble solid.

| | | |
|---|---|---|
| % Cl | Calc., 8.42 | Found, 6.46. |
| Cl/N | Calc., 0.51 | Found, 0.41 |
| C/N | Calc., 2.82 | Found, 2.92 |

### Example XV

### Poly[imino[1-[[[[3-hydroxypropyl]amino]carbonyl]methyl]-2-oxo-1,2-ethanediyl]imino[1-[[[[3-(trimethylammonio)propyl]amino]carbonyl]methyl]-2-oxo-1,2-ethanediyl chloride]]

In a manner similar to Example XIV, a copolymer of Formula 7 with R=OH, wherein (A):(B) = 1:2 can be prepared by reacting first 1.03g of 3-aminopropanol then 6.5g of N,N-dimethyl-1,3-propanediamine with 4.0 g polyanhydroaspartic acid, followed by quaternization.

### Example XVI

**Poly[imino[1-[[[[3-(pyridinium-1-yl)propyl]amino]carbonyl]methyl-2-oxo-1,2-ethanediyl chloride]]**

To a solution of 11.0g of 3-aminopropylpyridinium chloride hydrochloride in 10 mL H₂O and 40 mL of DMF are added in succession 8.0 mL of triethylamine and 2.0g of polyanhydroaspartic acid The reaction mixture is stirred for 72 h at room temperature then precipitated with ether. The precipitate is dissolved in a minimum amount of methanol and reprecipitated with ether. The precipitate is dissolved in 500 mL of H₂O, dialyzed and lyophilized to afford the water soluble title compound.

| | | |
|---|---|---|
| % Cl | Calc., 13.14 | Found, 8.81. |
| Cl/N | Calc., 0.97 | Found, 0.60 |
| C/N | Calc., 3.94 | Found, 3.44 |

### Example XVII

### Poly[imino[1-[2-[[[3-(trimethylammonio)propyl]amino]carbonyl]ethyl]-2-oxo-1,2-ethanediyl chloride]]

2.0g of poly (γ-methyl L-glutamate) is suspended in 30 mL of N,N-dimethyl-1,3-propanediamine and stirred under a nitrogen atmosphere for 5 days at 80°C, then precipitated with ether. The precipitate is dissolved in 100 mL of DMF, and to this solution is added 8.8g of dimethyl sulfate, then after 20 minutes 9.7g of potassium carbonate. The mixture is stirred 18 h at room temperature and the mixture is poured into ether. The solids are dissolved in 800 mL of H₂O and dialyzed until the effluent is pH=8.5. The aqueous solution is acidified with 25 mL of 6N hydrochloric acid, dialyzed until the effluent is pH=3, reacidified with 25 mL of 6N hydrochloric acid and dialyzed until the effluent is pH=6. The solution is concentrated to 100 mL and lyophilized to afford the water soluble title compound.

| | | |
|---|---|---|
| % Cl | Calc, 13.44 | Found, 10.71. |
| Cl/N | Calc., 0.84 | Found, 0.66 |
| C/N | Calc., 3.14 | Found, 3.01 |

### Example XVIII

### Poly[imino[1-[[[[3-(trimethylammonio)propyl]amino]carbonyl]methyl]-2-oxo-1,2-ethandiyl chloride]], [R], crosslinked with 0.2 0.5(-CH₂CH=CHCH₂-)

3.5g of polyanhydroaspartic acid is dissolved in 30 mL of anhydrous DMF and added dropwise to 9.2g of N,N-dimethyl-1,3-propanediamine while keeping the temperature of the reaction mixture below 30°C. The mixture is stirred under a nitrogen atmosphere for 18 h at room temperature, then precipitated with a mixture of ether and petroleum ether. The precipitate is dissolved in a minimum amount of methanol and reprecipitated with ether. The precipitate is dissolved in 500 mL H₂O, dialyzed, and lyophilized to afford 4.7g of an amber solid. This solid is dissolved in 50 mL DMF, and to this solution is added 0.44g of cis-1,4-dichloro-2-butene. The mixture is stirred for 4 h at 50°C, then 5.0 mL of dimethyl sulfate and 7.0g of potassium carbonate are added and the mixture is stirred 18 h at 50°C. The reaction mixture is then cooled to room temperature, filtered and precipitated with ether. The precipitate is dissolved in 500 mL H₂O and acidified with 25 mL of concentrated hydrochloric acid The solution is dialyzed as per Ex.I until the effluent is pH=3. An additional 25 mL of concentrated hydrochloric acid is added and the solution is redialyzed until the effluent is pH=6. The solution is concentrated by dialysis to 100 mL and lyophilized to afford the water soluble title compound.

| | | |
|---|---|---|
| % Cl | Calc., 14.06 | Found, 11.27 |
| Cl/N | Calc., 2.91 | Found, 2.98 |
| C/N | Calc., 0.84 | Found, 0.77 |

## Claims

1. A compound of the following Formula I: 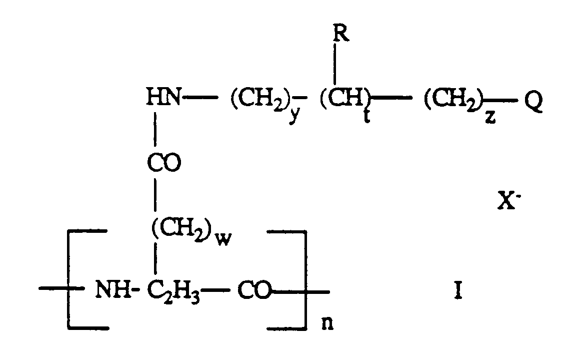 wherein
w is the integer 0 or 1;
y is the integer 1-6;
z is the integer 0-3;
t is the integer 0 or 1;
Q is 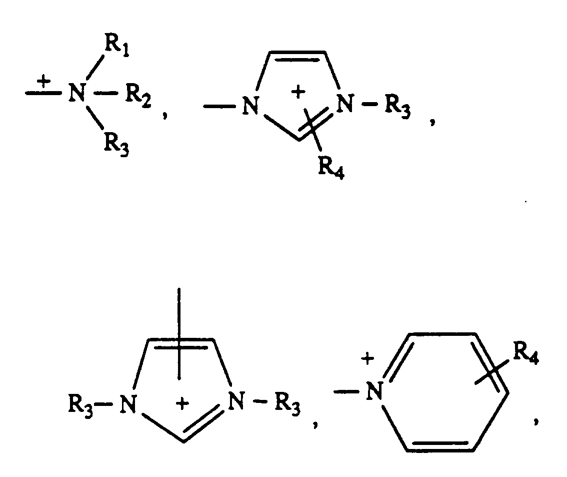 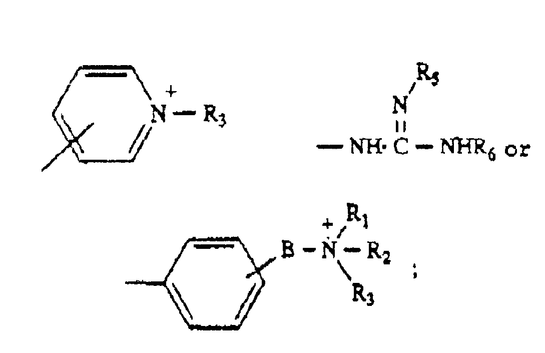
R is hydrogen, C₁-C₄ straight or branched alkyl, phenyl or when taken together with R₁ forms a saturated monoheterocyclic ring of from 5 to 6 members;
R₁,R₂ are the same or independently C₁-C₁₀ straight or branched chain alkyl which may be substituted by up to 3 substituents selected from 1 to 2 hydroxyl groups and one 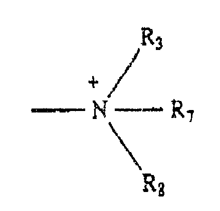 group, or when taken together form a saturated heterocyclic ring of from 5 to 6 members which may contains a 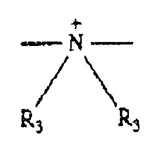 or an -O- linkage;
R₃ is methyl, ethyl benzyl which may be substituted by up to 3 substituents selected from hydroxyl, halogen, methoxy and C₁-C₆ straight or branched chain alkyl, 1-naphthyl or 2-naphthyl:
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which mayy be substituted by one hydroxyl group or when taken together with an -O- linkage form a morpholine ring;
B is a direct link or a C₁-C₄ straight or branched chain alkyl;
n is an integer in the range from about 50 to 500; and
X⁻ is an anion forming a pharmaceutically acceptable salt;
with the provisos that:
when R is hydrogen and Q is 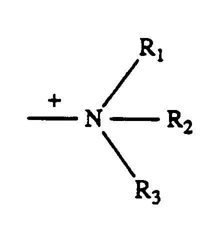 then R₃ cannot be methyl or ethyl when,
a) one or both of R₁ and R₂ is a C₁-C₁₀ straight or branched chain unsubstituted alkyl,
b) R₁ and R₂ together form a 6 membered mono or dihetero-oxygen containing cyclic ring.

2. A compound of claim 1 where w is the integer 0.

3. A compound of claim 2 where n is in the range of 100 to 500.

4. A compound of claim 3 where R is H, y is the integer 1, t is the integer 1 and z is the integer 0 or 1.

5. A compound of claim 4 where R₃ is methyl or benzyl.

6. A compound of claim 3 which is poly[imino[1-[[[[4-[(trimethylammonio)methyl]phenyl]methyl]amino]carbonyl]methyl]-2-oxo-1,2-ethanediyl chloride]].

7. A compound of Claim 3 which is poly[imino[1-[[[[4-[(aminoiminomethyl)amino]butyl]amino]carbonyl]methyl]-2-oxo-1,2-ethanediyl hydrochloride]].

8. A compound of claim 3 which is poly[imino[1-[[[[3-(1,3-dimethylimidazolium-2-yl)-propyl]amino]carbonyl]methyl]-2-oxo-1,2-ethanediyl chloride]].

9. A compound of claim 4 which is poly[imino[1-[[[[3-pyridinium-1-yl)propyl]amino]carbonyl]methyl]-2-oxo-1,2-ethanediyl chloride]].

10. A compound of claim 4 which is poly[imino[1-[[[[2-(1-methylpyridinium-2-yl)ethyl]amino]carbonyl]methyl]-2-oxo-1,2-ethanediyl chloride]].

11. A compound of claim 4 which is poly[imino[1-[[[[2-(1,3-dimethylimidazolium-4-yl)ethyl]amino]carbonyl]methyl]-2-oxo-1,2-ethanediyl chloride]].

12. A compound of claim 4 which is poly[imino[1-[[[[4-(trimethylammonio)phenyl]methyl]amino]carbonyl]methyl]-2-oxo-1,2-ethanediyl chloride]].

13. A compound of claim 4 which is poly[imino[1-[[[[3-[(aminoiminomethyl)amino)propyl]amino]carbonyl]methyl]-2-oxo-1,2-ethanediyl hydrochloride]].

14. A compound of claim 4 which is poly[imino[1-[[[[2-[(aminoiminomethyl)amino]ethyl]amino]carbonyl]methyl]-2-oxo-1,2-ethanediyl hydrochloride]].

15. A compound of claim 5 which is poly[imino[1-[[[[2-[[3-(trimethylammonio)propyl]dimethylammonio]ethyl]amino]carbonyl]methyl]-2-oxo- 1,2-ethanediyl dichloride]].

16. A compound of claim 5 which is poly[imino[1-[[[[2-[[2-(trimethylammonio)ethyl]dimethylammonio]ethyl]amino]carbonyl]-methyl]-2-oxo-1,2-ethandiyl dichloride]].

17. A compound of claim 5 which is poly[imino[1-[[[[2-[1-methylpyrrolidinium-1-yl]ethyl]amino]carbonyl]methyl]-2-oxo-1,2-ethanediyl chloride]].

18. A compound of claim 5 which is poly[imino[1-[[[[2-[bis(2-hydroxyethyl)methylammonio]-ethyl]amino]carbonyl]methyl]-2-oxo-1,2-ethanediyl chloride]].

19. A compound of claim 5 which is poly[imino[1-[[[[2-[dimethyl(phenylmethyl)ammonio]-ethyl]amino]carbonyl]methyl]-2-oxo-1,2-ethanediyl chloride]].

20. A compound of claim 5 which is poly[imino[1-[[[[3-[dimethyl(phenylmethyl)ammonio]propyl]amino]carbonyl]methyl]-2-oxo-1,2-ethanediyl chloride]].

21. A copolymer having monomeric units of the following formula [NH-(C₂H₃)A-CO] where A is (CH₂)_{w}-CO-HN-(CH₂)_{y}-(CH₂)ₜR-(CH₂)_{z}-Q and where
w is the integer 0 or 1;
y is the integer 1-6;
z is the integer 0-3;
t is the integer 0 or 1;
Q is 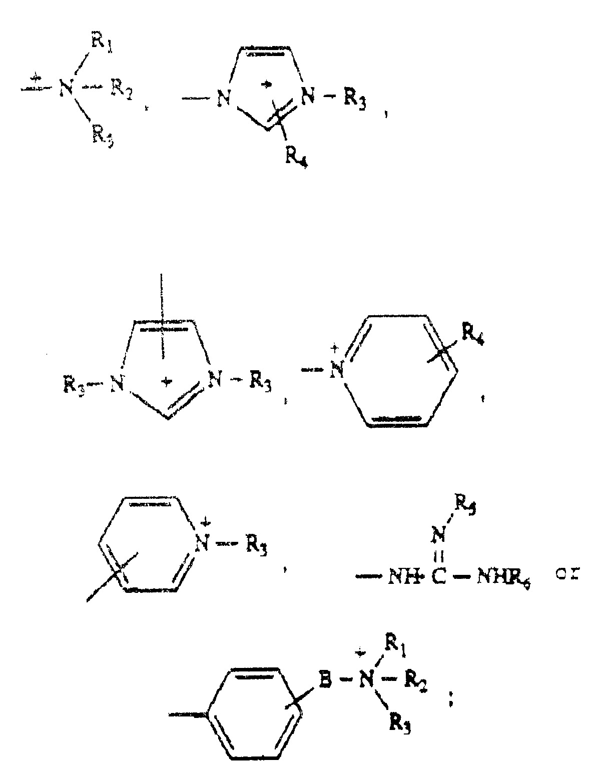
R is hydrogen, C₁-C₄ straight or branched alkyl, phenyl or when taken together with R₁ forms a saturated monoheterocyclic ring of from 5 to 6 members;
R₁, R₂ are the same or independently C₁-C₁₀ straight or branched chain alkyl which may be substituted by up to 3 substituents selected from 1 to 2 hydroxyl groups and one 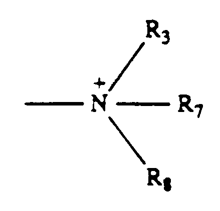 group, or when taken together form a saturated heterocyclic ring of from 5 to 6 members which may contain a 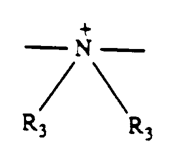 or -O- linkage;
R₃ is methyl, ethyl, benzyl which may be substituted by up to 3 substituents selected from hydroxyl methoxy, halogen and C₁-C₆straight or branched chain alkyl, 1-naphthyl or 2-naphthyl;
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which may be substituted by one hydroxyl group or when taken together with an -O- linkage form the morpholine moiety ring;
B is a direct link or a C₁-C₄ straight or branched chain alkyl;
X⁻ is an anion forming a pharmaceutically acceptable salt ;
and where said monomeric units are different and wherein the overall number of said units is in the range of from about 50 to 500.

22. The use of a compound of the following structural formula II: 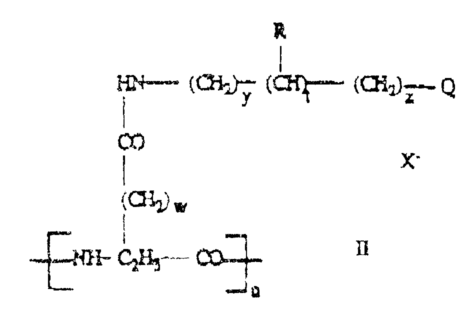 wherein
w is the integer 0 or 1;
y is the integer 1-6;
z is the integer 0-3;
t is the integer 0 or 1;
Q is 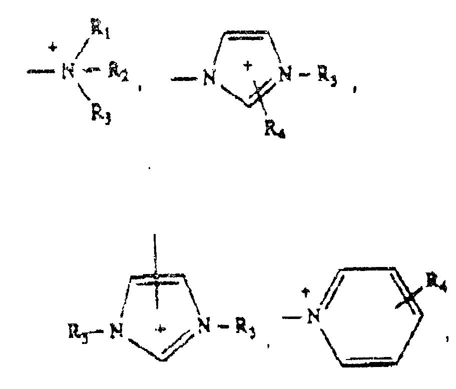 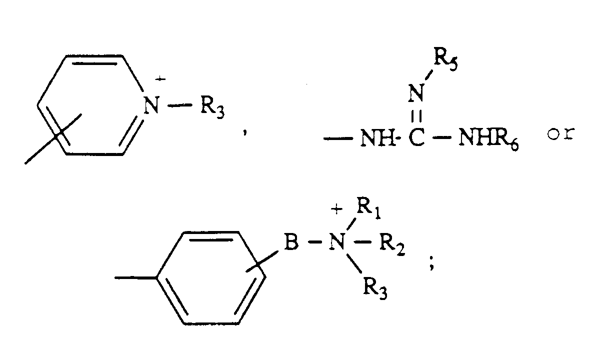
R is hydrogen, C₁-C₄ straight or branched alkyl, phenyl or when taken together with R₁ forms a saturated monoheterocyclic ring of from 5 to 6 members;
R₁,R₂ are the same or independently C₁-C₁₀ straight or branched chain alkyl which may be substituted by up to 3 substituents selected from 1 to 2 hydroxyl groups and one 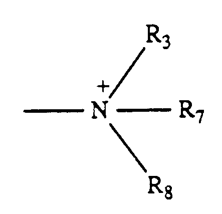 group, or when taken together form a saturated heterocyclic ring of from 5 to 6 members which may contains a 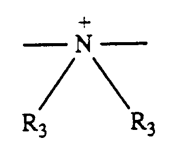 or an -O- linkage;
R₃ is methyl, ethyl, benzyl which may be substituted by up to 3 substituents selected from hydroxyl, halogen, methoxy and C₁-C₆ straight or branched chain alkyl, 1-naphthyl or 2-naphthyl;
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which may be substituted by one hydroxyl group or when taken together with an -O- linkage form a morpholine ring:
B is a direct link or a C₁-C₄ straight or branched chain alkyl,
n is an integer in the range from about 50 to 500; and
X⁻ is an anion forming a pharmaceutically acceptable salt for the manufacture of a pharmaceutical composition for lowering lipid activity in a mammal.

23. The use of a compound of the following structural formula II: 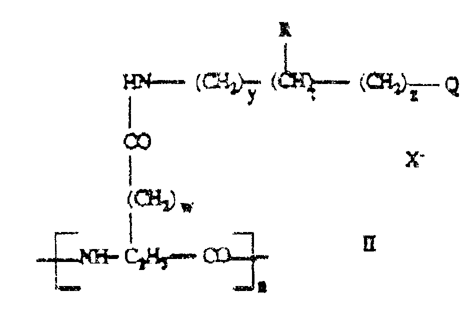 wherein
w is the integer 0 or 1;
y is the integer 1-6;
z is the integer 0-3;
t is the integer 0 or 1;
Q is 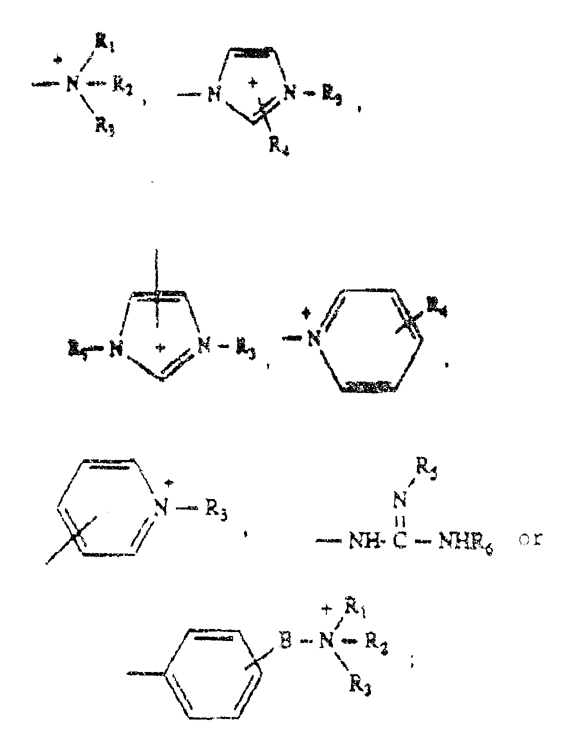
R is hydrogen, C₁-C₄ straight or branched alkyl, phenyl or when taken together with R₁ forms a saturated monoheterocyclic ring of from 5 to 6 members;
R₁,R₂ are the same or independently C₁-C₁₀ straight or branched chain alkyl which may be substituted by up to 3 substituents selected from 1 to 2 hydroxyl groups and one 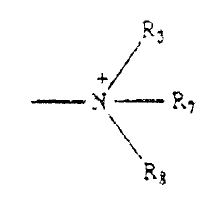 group, or when taken together form a saturated heterocyclic ring of from 5 to 6 members which may contains a 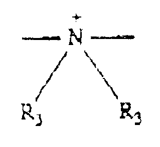 or an -O- linkage;
R₃ is methyl, ethyl, benzyl which may be substituted by up to 3 substituents selected from hydroxyl, halogen, methoxy and C₁-C₆ straight or branched chain alkyl, 1-naphthyl or 2-naphthyl;
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which mayy be substituted by one hydroxyl group or when taken together with an -O- linkage form a morpholine ring;
B is a direct link or a C₁-C₄ straight or branched chain alkyl;
n is an integer in the range from about 50 to 500; and
X⁻ is an anion forming a pharmaceutically acceptable salt
for the manufacture of a pharmaceutical composition for dissolving, delaying progression and/or blocking the formation of gallstones in humans.

24. A pharmaceutical composition comprising a lipid lowering effective amount of a compound of the following Formula II: 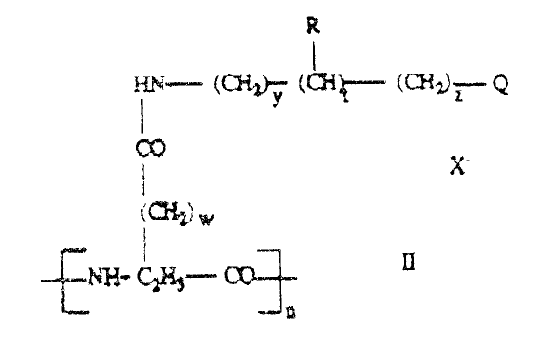 wherein
w is the integer 0 or 1;
y is the integer 1-6;
z is the integer 0-3;
t is the integer 0 or 1;
Q is 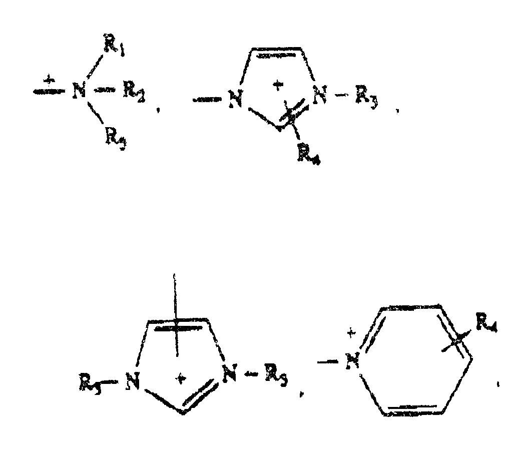 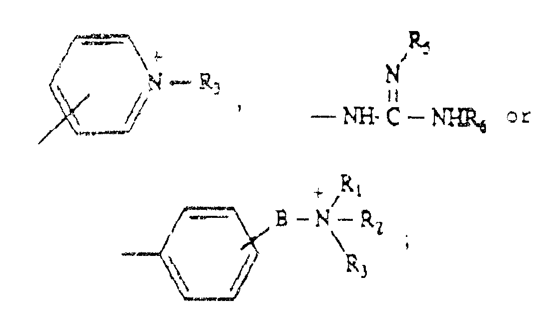
R is hydrogen, C₁-C₄ straight or branched alkyl , phenyl or when taken together with R₁ forms a saturated monoheterocyclic ring of from 5 to 6 members;
R₁, R₂ are the same or independently C₁-C₁₀ straight or branched chain alkyl which may be substituted by up to 3 substituents selected from 1 to 2 hydroxyl groups and one 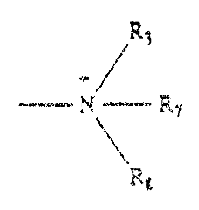 group, or when taken together from a saturated heterocyclic ring of from 5 to 6 members which may contain a 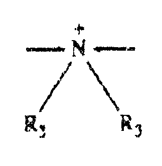 or -O- linkage;
R₃ is methyl, ethyl, benzyl which may substituted by up to 3 substituents selected from hydroxyl, methoxy, halogen and C₁-C₆straight or branched chain alkyl, 1-naphthyl or 2-naphthyl;
R₄ is a C₁-C₆ straight or branched chain alkyl which may be substituted by one hydroxyl group;
R₅, R₆ are the same or independently hydrogen, C₁-C₄ straight or branched chain alkyl or when taken together form a diheterocyclic ring of from 5 to 6 members;
R₇, R₈ are the same or independently C₁-C₆ straight or branched chain alkyl which may be substituted by one hydroxyl group or when taken together with an -O- linkage form the morpholine moiety ring;
B is a direct link or a C₁-C₄ straight or branched chain alkyl;
n is an integer in the range from about 50 to 500; and
X⁻ is an anion forming a pharmaceutically acceptable salt,
together with a non-toxic pharmaceutically acceptable carrier.

## Patentansprüche

1. Eine Verbindung der folgenden Formel I: 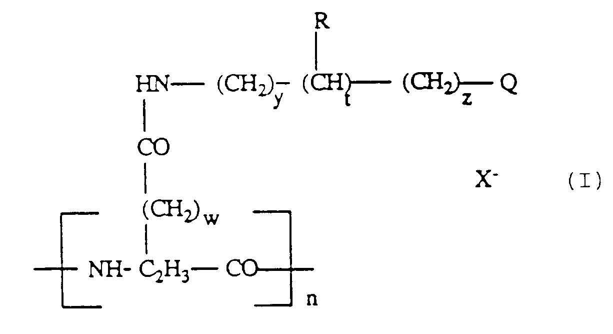 worin
w die ganze Zahl 0 oder 1 ist;
y die ganze Zahl 1-6 ist;
z die ganze Zahl 0-3 ist;
t die ganze Zahl 0 oder 1 ist;
Q 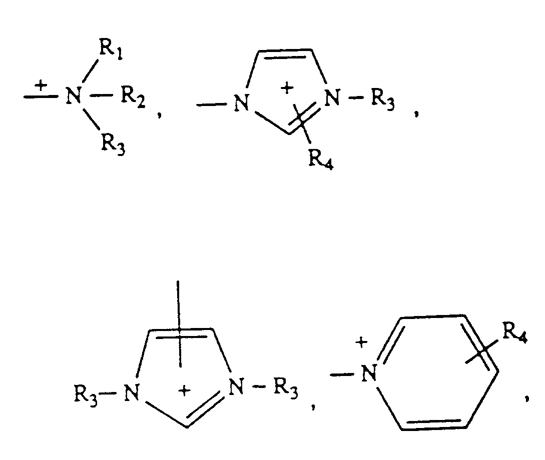 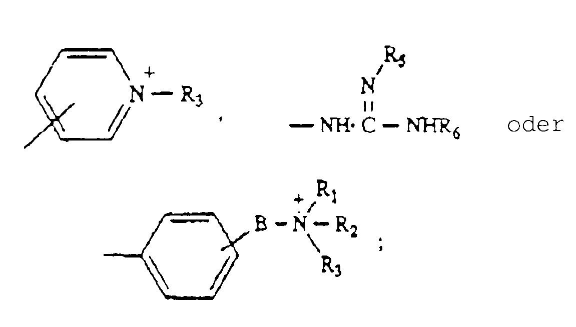
bedeutet,
R bedeutet Wasserstoff, eine gerade oder verzweigte C₁-C₄-Alkylkette, Phenyl oder wenn mit R₁ zusammengenommen, wird ein gesättigter mono-heterocyclischer Ring mit 5 bis 6 Ringglieder gebildet;
R₁, R₂ sind die Gleichen oder voneinander unabhängige gerade oder verzweigte C₁-C₁₀-Alkylketten, die mit bis zu drei Substituenten, ausgewählt aus 1 bis zwei Hydroxylgruppen und einer 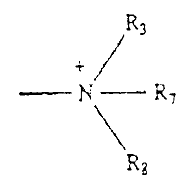 Gruppe substituiert sein können, oder wenn Sie zusammengenommen werden, einen gesättigten heterocyclischen Ring von zwischen 5 bis 6 Ringgliedern bilden, der eine 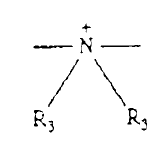 Gruppe oder ein -O-Bindeglied enthalten kann;
R₃ ist Methyl, Ethyl, Benzyl die mit bis zu drei Substituenten, ausgewählt aus Hydroxyl, Halogen, Methoxy und einer geraden oder verzweigten C₁-C₆-Alkylkette, 1-Naphthyl oder 2-Naphthyl, substituiert sein können;
R₄ ist eine gerade oder verzweigte C₁-C₆-Alkylkette die mit einer Hydroxylgruppe substituiert sein kann;
R₅, R₆ sind die Gleichen oder sind unabhängig voneinander Wasserstoff, eine gerade oder verzweigte C₁-C₄-Alkylkette, oder wenn sie zusammengenommen werden, bilden sie einen di-heterocyclischen Ring mit 5 bis 6 Ringglieder;
R₇, R₈ sind die Gleichen oder sind unabhängig voneinander eine gerade oder verzweigte C₁-C₆-Alkylkette, die mit einer Hydroxylgruppe substituiert sind, oder sie bilden einen Morpholinring, wenn sie mit einem -O-Bindeglied zusammengenommen werden;
B ist eine direkte Bindung oder eine gerade oder verzweigte C₁-C₄-Alkylkette;
n ist eine ganze Zahl im Bereich von etwa 50 bis 500;
X⁻ ist ein Anion, das ein pharmazeutisch annehmbares Salz bildet;
unter der Vorraussetzung, daß
wenn R Wasserstoff und Q 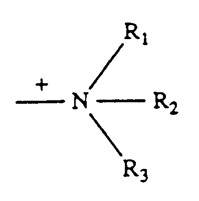 ist,
dann kann R₃ nicht Methyl oder Ethyl sein, wenn,
a) einer oder beide von R₁, R₂ eine gerade oder verzweigte unsubstituierte C₁-C₁₀-Alkylkette ist,
b) R₁, R₂ zusammen einen 6-gliedrigen mono- oder di-hetero-, Sauerstoff enthaltenden cyclischen Ring bilden.

2. Eine Verbindung gemäß Anspruch 1, wobei w die ganze Zahl 0 ist.

3. Eine Verbindung gemäß Anspruch 2, wobei n im Bereich von 100 bis 500 liegt.

4. Eine Verbindung gemäß Anspruch 3, wobei R Wasserstoff ist, y die ganze Zahl 1 ist, t die ganze Zahl 1 ist und z die ganze Zahl 0 oder 1 ist.

5. Eine Verbindung gemäß Anspruch 4, wobei R₃ Methyl oder Benzyl ist.

6. Eine Verbindung gemaß Anspruch 3, welche das Poly[imino[1-[[[[4-[(trimethylammonio)methyl]-phenyl]methyl]amino]carbonyl]methyl]-2-oxo-1,2-ethandiyl chlorid]] ist.

7. Eine Verbindung gemäß Anspruch 3, welche das Poly[imino[1-[[[[4-[(aminoiminomethyl)amino]-butyl]amino]carbonyl]methyl]-2-oxo-1,2-ethandiyl hydrochlorid]] ist.

8. Eine Verbindung gemäß Anspruch 3, welche das Poly[imino[1-[[[[3-(1,3-dimethylimidazoiium-2-yl)-propyl]amino]carbonyl]methyl]-2-oxo-1,2-ethandiyl chlorid]] ist.

9. Eine Verbindung gemäß Anspruch 4, welche das Poly[imino[1-[[[[3-pyridinium-1-yl)propyl]-amino]carbonyl]methyl]-2-oxo-1,2-ethandiyl chlorid]] ist.

10. Eine Verbindung gemäß Anspruch 4, welche das Poly[imino[1-[[[[2-(1-methylpyridinium-2-yl)ethyl]amino]carbonyl]methyl]-2-oxo-1,2-ethandiyl chlorid]] ist.

11. Eine Verbindung gemäß Anspruch 4, welche das Poly[imino[1-[[[[2-(1,3-dimethylimidazolium-4-yl)ethyl]-amino]carbonyl]methyl]-2-oxo-1,2-ethandiyl chlorid]] ist.

12. Eine Verbindung gemäß Anspruch 4, welche das Poly[imino[1-[[[[4-(trimethylammonio)phenyl]methyl]amino]carbonyl]methyl]-2-oxo-1,2-ethandiyl chlorid]] ist.

13. Eine Verbindung gemäß Anspruch 4, welche das Poly[imino[1-[[[[3-[(aminoiminomethyl)amino)propyl]amino]carbonyl]methyl]-2-oxo-1,2-ethandiyl hydrochlorid]] ist.

14. Eine Verbindung gemäß Anspruch 4, welche das Poly[imino[1-[[[[2-[(aminoiminomethyl)amino)ethyl]amino]carbonyl]methyl]-2-oxo-1,2-ethandiyl hydrochlorid]] ist.

15. Eine Verbindung gemäß Anspruch 5, welche das Poly[imino[1-[[[[2-[[3-(trimethylammonio)propyl]dimethylammonio]ethyl]amino]carbonyl]methyl]-2-oxo-1,2-ethandiyl dichlorid]] ist.

16. Eine Verbindung gemäß Anspruch 5, welche das Poly[imino[1-[[[[2-[[2-(trimethylammonio)ethyl]-dimethylammonio]ethyl]amino]carbonyl]methyl]-2-oxo-1,2-ethandiyl dichlorid]] ist.

17. Eine Verbindung gemäß Anspruch 5, welche das Poly[imino[1-[[[[2-[1-(methylpyrrolidinium-1-yl]-ethyl]amino]carbonyl]methyl]-2-oxo-1,2-ethandiyl chlorid]] ist.

18. Eine Verbindung gemäß Anspruch 5, welche das Poly[imino[1-[[[[2-[bis(2-hydroxyethyl)methylammonio]ethyl]amino]carbonyl]methyl]-2-oxo-1,2-ethandiyl chlorid]] ist.

19. Eine Verbindung gemäß Anspruch 5, welche das Poly[imino[1-[[[[2-[dimethyl(phenylmethyl)ammonio]ethyl]-amino]carbonyl]methyl]-2-oxo-1,2-ethandiyl chlorid]] ist.

20. Eine Verbindung gemäß Anspruch 5, welche das Poly[imino[1-[[[[3-[dimethyl(phenylmethyl)ammonio]propyl]amino]carbonyl]methyl]-2-oxo-1,2-ethandiyl chlorid]] ist.

21. Ein Co-Polymer, das die Monomereinheit der folgenden Formel besitzt: [NH-(C₂H₃)A-CO], wobei A gleich (CH₂)_{w}-CO-NH-(CH₂)_{y}-(CH₂)ₜR-(CH₂)_{z}-Q ist und wobei
w die ganze Zahl 0 oder 1 ist;
y die ganze Zahl 1-6 ist;
z die ganze Zahl 0-3 ist;
t die ganze Zahl 0 oder 1 ist;
Q 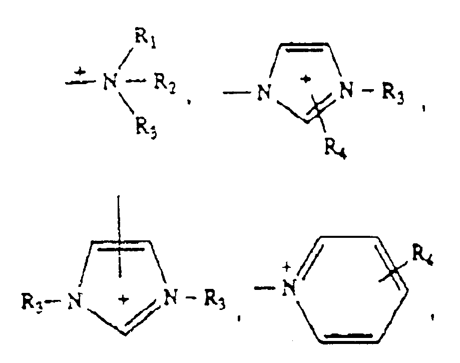 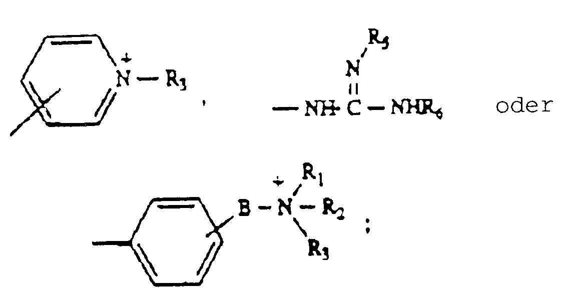
bedeutet,
R bedeutet Wasserstoff, eine gerade oder verzweigte C₁-C₄-Alkylkette, Phenyl oder wenn mit R₁ zusammengenommen, wird ein gesättigter mono-heterocyclischer Ring mit 5 bis 6 Ringglieder gebildet;
R₁, R₂ sind die Gleichen oder voneinander unabhängige gerade oder verzweigte C₁-C₁₀-Alkylketten, die mit bis zu drei Substituenten, ausgewählt aus 1 bis zwei Hydroxylgruppen und einer 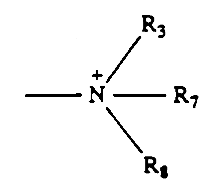 Gruppe substituiert sein können, oder wenn Sie zusammengenommen werden einen gesättigten heterocyclischen Ring von zwischen 5 bis 6 Ringglieder bilden, der eine 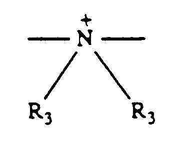 Gruppe oder ein -O-Bindeglied enthalten kann;
R₃ ist Methyl, Ethyl, Benzyl die mit bis zu drei Substituenten, ausgewählt aus Hydroxyl, Halogen, Methoxy und einer geraden oder verzweigten C₁-C₆-Alkylkette, 1-Naphthyl oder 2-Naphthyl, substituiert sein können;
R₄ ist eine gerade oder verzweigte C₁-C₆-Alkylkette, die mit einer Hydroxylgruppe substituiert sein kann;
R₅, R₆ sind die Gleichen oder sind unabhängig voneinander Wasserstoff, eine gerade oder verzweigte C₁-C₄-Alkylkette, oder wenn sie zusammengenommen werden, bilden sie einen di-heterocyclischen Ring mit 5 bis 6 Ringglieder;
R₇, R₈ sind die Gleichen oder sind unabhängig voneinander eine gerade oder verzweigte C₁-C₆-Alkylkette, die mit einer Hydroxylgruppe substituiert ist oder sie bilden einen Morpholinring, wenn sie mit einem -O-Bindeglied zusammengenommen werden;
B ist eine direkte Bindung oder eine gerade oder verzweigte C₁-C₄-Alkylkette;
X⁻ ist ein Anion, das ein pharmazeutisch annehmbares Salz bildet;
und wobei die besagten Monomereinheiten verschieden sind und wobei die Gesamtzahl der besagten Einheiten im Bereich von etwa 50 bis 500 liegt.

22. Verwendung einer Verbindung der folgenden Struktur II: 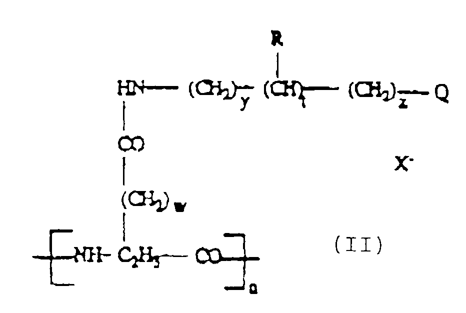 worin
w die ganze Zahl 0 oder 1 ist;
y die ganze Zahl 1-6 ist;
z die ganze Zahl 0-3 ist;
t die ganze Zahl 0 oder 1 ist;
Q 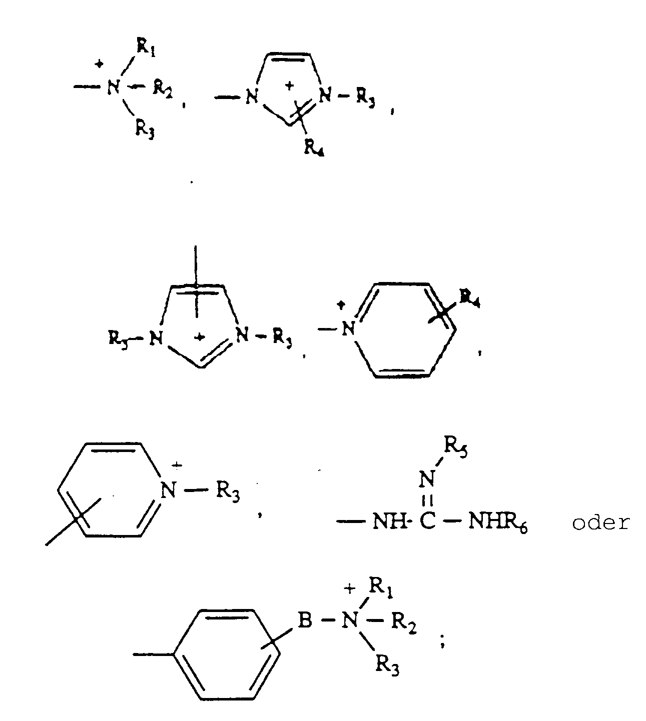
bedeutet,
R bedeutet Wasserstoff, eine gerade oder verzweigte C₁-C₄-Alkylkette, Phenyl oder wenn mit R₁ zusammengenommen, wird ein gesättigter mono-heterocyclischer Ring mit 5 bis 6 Ringglieder gebildet;
R₁, R₂ sind die Gleichen oder voneinander unabhängige gerade oder verzweigte C₁-C₁₀-Alkylketten, die mit bis zu drei Substituenten, ausgewählt aus 1 bis zwei Hydroxylgruppen und einer 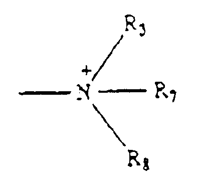 Gruppe substituiert sein können, oder wenn Sie zusammengenommen werden einen gesättigten heterocyclischen Ring von zwischen 5 bis 6 Ringglieder bilden, der eine 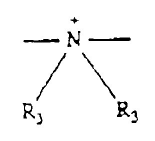 Gruppe oder ein -O-Bindeglied enthalten kann;
R₃ ist Methyl, Ethyl, Benzyl die mit bis zu drei Substituenten, ausgewählt aus Hydroxyl, Halogen, Methoxy und einer gerade oder verzweigten C₁-C₆-Alkylkette, 1-Naphthyl oder 2-Naphthyl, substituiert sein können;
R₄ ist eine gerade oder verzweigte C₁-C₆-Alkylkette die mit einer Hydroxylgruppe substituiert sein kann;
R₅, R₆ sind die Gleichen oder sind unabhängig voneinander Wasserstoff, eine gerade oder verzweigte C₁-C₄-Alkylkette, oder wenn sie zusammengenommen werden, bilden sie einen di-heterocyclischen Ring mit 5 bis 6 Ringglieder;
R₇, R₈ sind die Gleichen oder sind unabhängig voneinander eine gerade oder verzweigte C₁-C₄-Alkylkette, die mit einer Hydroxylgruppe substituiert sind oder sie bilden einen Morpholinring, wenn sie mit einem -O-Bindeglied zusammengenommen werden;
B ist eine direkte Bindung oder eine gerade oder verzweigte C₁-C₄-Alkylkette;
n ist eine ganze Zahl im Bereich von etwa 50 bis 500;
X⁻ ist ein Anion, das ein pharmazeutisch annehmbares Salz, für die Herstellung einer pharmazeutischen Zusammensetzung zur Erniedrigung der Lipid-Aktivität in einem Säuger, bildet.

23. Die Verwendung einer Verbindung der folgenden Strukturformel II: 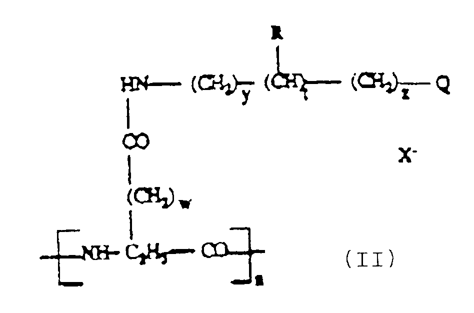 worin
w die ganze Zahl 0 oder 1 ist;
y die ganze Zahl 1-6 ist;
z die ganze Zahl 0-3 ist;
t die ganze Zahl 0 oder 1 ist;
Q 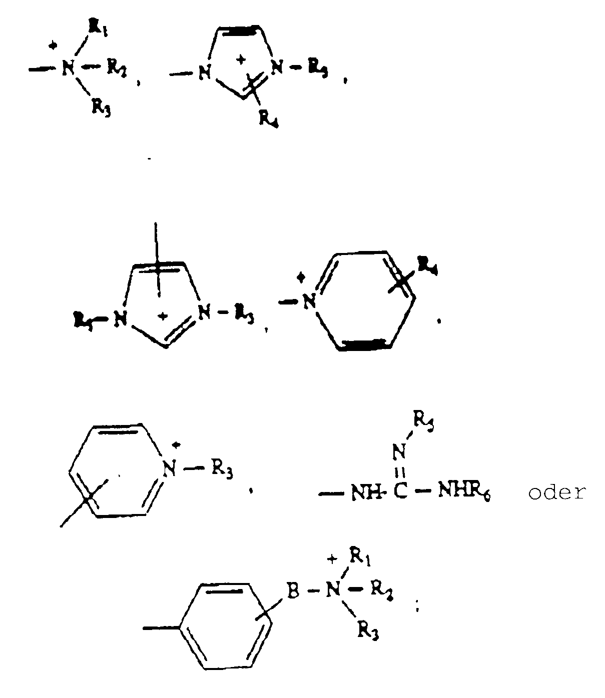
bedeutet,
R bedeutet Wasserstoff, eine gerade oder verzweigte C₁-C₄-Alkylkette, Phenyl oder wenn mit R₁ zusammengenommen, wird ein gesättigter mono-heterocyclischer Ring mit 5 bis 6 Ringglieder gebildet;
R₁, R₂ sind die Gleichen oder voneinander unabhängige gerade oder verzweigte C₁-C₁₀-Alkylketten, die mit bis zu drei Substituenten, ausgewählt aus 1 bis zwei Hydroxylgruppen und einer 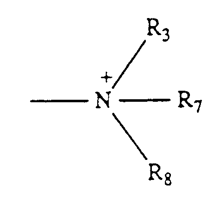 Gruppe substituiert sein können, oder wenn Sie zusammengenommen werden einen gesättigten heterocyclischen Ring von zwischen 5 bis 6 Kettengliedern bilden, der eine 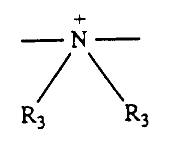 Gruppe oder ein -O-Bindeglied enthalten kann;
R₃ ist Methyl, Ethyl, Benzyl die mit bis zu drei Substituenten, ausgewählt aus Hydroxyl, Halogen, Methoxy und einer geraden oder verzweigten C₁-C₆-Alkylkette, 1-Naphthyl oder 2-Naphthyl, substituiert sein können;
R₄ ist eine gerade oder verzweigte C₁-C₆-Alkylkette die mit einer Hydroxylgruppe substituiert sein kann;
R₅, R₆ sind die Gleichen oder sind unabhängig voneinander Wasserstoff, eine gerade oder verzweigte C₁-C₄-Alkylkette, oder wenn sie zusammengenommen werden, bilden sie einen di-heterocyclischen Ring von 5 bis 6 Ringglieder;
R₇, R₈ sind die Gleichen oder sind unabhängig voneinander eine gerade oder verzweigte C₁-C₄-Alkylkette, die mit einer Hydroxylgruppe substituiert sind oder sie bilden einen Morpholinring, wenn sie mit einem -O-Bindeglied zusammengenommen werden;
B ist eine direkte Bindung oder eine gerade oder verzweigte C₁-C₄-Alkylkette;
n ist eine ganze Zahl im Bereich von etwa 50 bis 500;
X⁻ ist ein Anion, das ein pharmazeutisch annehmbares Salz, für die Herstellung einer pharmazeutischen Zusammensetzung zur Auflösung, Verzögerung des Voranschreitens und/oder die Blockierung der Bildung von Gallensteine bei Menschen, bildet.

24. Eine pharmazeutische Zusammensetzung, umfassend eine ausreichende Menge einer Lipid-erniedrigende Verbindung der folgenden Formel II: 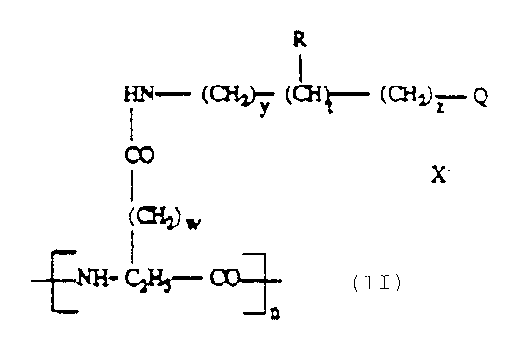 worin
w die ganze Zahl 0 oder 1 ist;
y die ganze Zahl 1-6 ist;
z die ganze Zahl 0-3 ist;
t die ganze Zahl 0 oder 1;
Q 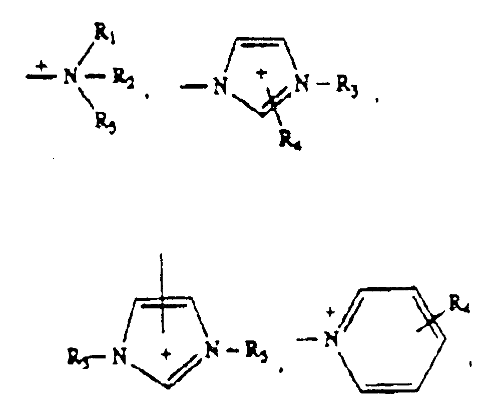 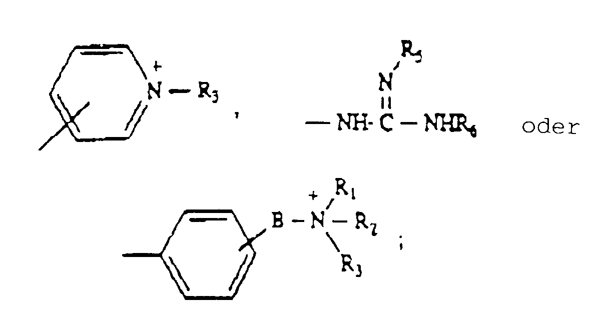
bedeutet,
R bedeutet Wasserstoff, eine gerade oder verzweigte C₁-C₄-Alkylkette, Phenyl oder wenn mit R₁ zusammengenommen, wird ein gesättigter mono-heterocyclischer Ring mit 5 bis 6 Ringglieder gebildet;
R₁, R₂ sind die Gleichen oder voneinander unabhängige gerade oder verzweigte C₁-C₁₀-Alkylketten, die mit bis zu drei Substituenten, ausgewählt aus 1 bis zwei Hydroxylgruppen und einer 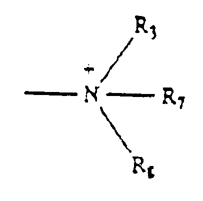 Gruppe substituiert sein können, oder wenn Sie zusammengenommen werden einen gesättigten heterocyclischen Ring von zwischen 5 bis 6 Kettengliedern bilden, der eine 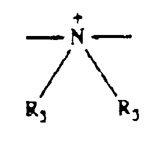 Gruppe oder ein -O-Bindeglied enthalten;
R₃ ist Methyl, Ethyl, Benzyl die mit bis zu drei Substituenten, ausgewählt aus Hydroxyl, Halogen, Methoxy und einer geraden oder verzweigten C₁-C₆-Alkylkette, 1-Naphthyl oder 2-Naphthyl, substituiert sein können;
R₄ ist eine gerade oder verzweigte C₁-C₆-Alkylkette die mit einer Hydroxylgruppe substituiert sein kann;
R₅, R₆ sind die Gleichen oder sind unabhängig voneinander Wasserstoff, eine gerade oder verzweigte C₁-C₄-Alkylkette, oder wenn sie zusammengenommen werden, bilden sie einen di-heterocyclischen Ring von 5 bis 6 Ringglieder;
R₇, R₈ sind die Gleichen oder sind unabhängig voneinander eine gerade oder verzweigte C₁-C₄-Alkylkette, die mit einer Hydroxylgruppe substituiert ist oder wenn sie mit einem -O-Bindeglied zusammengenommen werden bilden sie einen Morpholinring,;
B ist eine direkte Bindung oder eine gerade oder verzweigte C₁-C₄-Alkylkette;
n ist eine ganze Zahl im Bereich von etwa 50 bis 500;
X⁻ ist ein Anion, das ein pharmazeutisch annehmbares Salz bildet;
zusammen mit einem nicht-toxischen pharmazeutisch annehmbaren Carrier (Träger).

## Revendications

1. Un composé de la Formule I suivante : 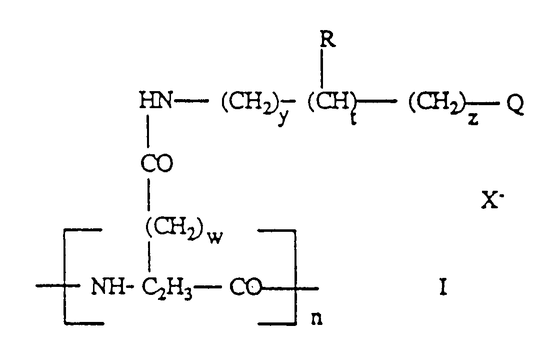 dans laquelle
w est le nombre entier 0 ou 1 ;
y est un nombre entier de 1 à 6 ;
z est un nombre entier de 0 à 3 ;
t est le nombre entier 0 ou 1 ;
Q est 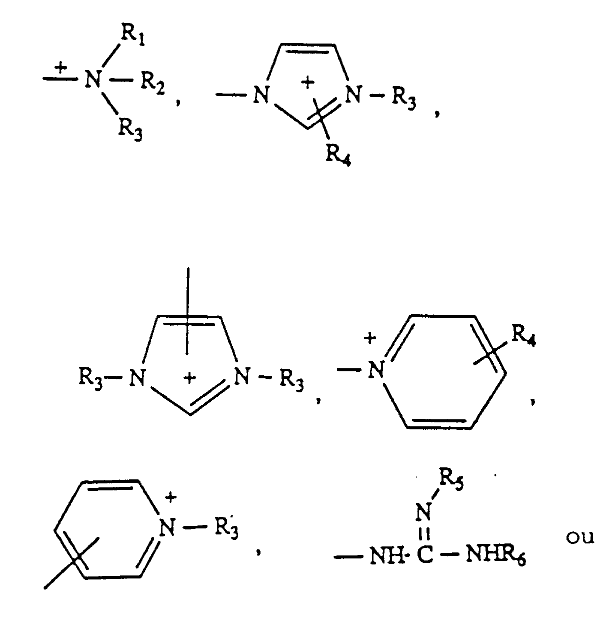 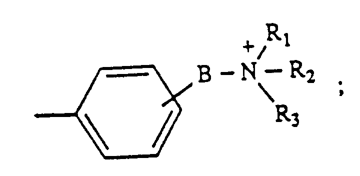
R est l'hydrogène, un groupe alkyle en C₁-C₄ à chaîne droite ou ramifiée, phényle ou, s'il est pris avec R₁, forme un monohétérocycle saturé penta- ou hexagonal ;
R₁, R₂ sont chacun identiquement ou indépendamment un groupe alkyle en C₁-C₁₀ à chaîne droite ou ramifiée qui peut être substitué par un maximum de 3 substituants choisis parmi 1 ou 2 groupes hydroxyle et un groupe 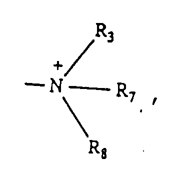 ou bien, s'ils sont pris ensemble, forment un hétérocycle saturé penta- ou hexagonal qui peut contenir une liaison 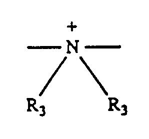 ou -O- ;
R₃ est un groupe méthyle, éthyle, benzyle qui peut être substitué par un maximum de 3 substituants choisis parmi les groupes hydroxyle, halogéno, méthoxy et alkyle en C₁-C₆ à chaîne droite ou ramifiée, 1-naphtyle ou 2-naphtyle ;
R₄ est un groupe alkyle en C₁-C₆ à chaîne droite ou ramifiée qui peut être substitué par un groupe hydroxyle ;
R₅, R₆ sont chacun identiquement ou indépendamment l'hydrogène, un groupe alkyle en C₁-C₄ à chaîne droite ou ramifiée, ou bien, s'ils sont pris ensemble, forment un dihétérocycle penta- ou hexagonal ;
R₇, R₈ sont chacun identiquement ou indépendamment un groupe alkyle en C₁-C₆ à chaîne droite ou ramifiée qui peut être substitué par un groupe hydroxyle, ou bien, s'ils sont pris ensemble avec une liaison -O-, forment un cycle de morpholine ;
B est une liaison directe ou un groupe alkyle en C₁-C₄ à chaîne droite ou ramifiée ;
n est un nombre entier compris dans l'intervalle d'environ 50 à 500 ; et
X⁻ est un anion formateur de sel pharmaceutiquement acceptable.
avec les conditions que :
si R est l'hydrogène et Q est 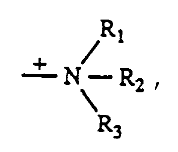 alors R₃ ne peut pas être un groupe méthyle ou éthyle ;
si
a) l'un ou chacun de R₁ et R₂ est un groupe alkyle en C₁-C₁₀ non substitué à chaîne droite ou ramifiée,
b) R₁ et R₂ forment ensemble un mono- ou dihétérocycle oxygéné hexagonal.

2. Un composé de la revendication 1, dans lequel w est le nombre entier 0.

3. Un composé de la revendication 2, dans lequel n est compris dans l'intervalle de 100 à 500.

4. Un composé de la revendication 3, dans lequel R est H, y est le nombre entier 1, t est le nombre entier 1 et z est le nombre entier 0 ou 1.

5. Un composé de la revendication 4, dans lequel R₃ est le groupe méthyle ou benzyle.

6. Un composé de la revendication 3, qui est le poly[imino[chlorure de 1-[[[[4-[(triméthylammonio)méthyl]phényl]méthyl]amino]carbonyl]méthyl]-2-oxo-1,2-éthanediyle]].

7. Un composé de la revendication 3, qui est le poly[imino[chlorhydrate de 1-[[[[4-[(amino-iminométhyl)-amino]butyl]amino]carbonyl]méthyl]-2-oxo-1,2-éthanediyle]].

8. Un composé de la revendication 3, qui est le poly[imino[chlorure de 1-[[[[3-(1,3-diméthylimidazolium-2-yl)propyl]amino]carbonyl]méthyl]-2-oxo-1,2-éthanediyle]].

9. Un composé de la revendication 4, qui est le poly[imino[chlorure de 1-[[[[3-pyridinium-1-yl)propyl]amino]carbonyl]méthyl]-2-oxo-1,2-éthanediyle]].

10. Un composé de la revendication 4, qui est le poly[imino[chlorure de 1-[[[[2-(1-méthylpyridinium-2-yl)éthyl]amino]carbonyl]méthyl]-2-oxo-1,2-éthanediyle]].

11. Un composé de la revendication 4, qui est le poly[imino[chlorure de 1-[[[[2-(1,3-diméthylimidazolium-4-yl)éthyl]amino]carbonyl]méthyl-2-oxo-1,2-éthanediyle]].

12. Un composé de la revendication 4, qui est le poly[imino[chlorure de 1-[[[[4-(triméthylammonio)phényl]méthyl]amino]carbonyl]méthyl]-2-oxo-1,2-éthanediyle]].

13. Un composé de la revendication 4, qui est le poly[imino[chlorhydrate de 1-[[[[3-[(amino-iminométhyl)-amino)propyl]amino]carbonyl]méthyl]-2-oxo-1,2-éthanediyle]].

14. Un composé de la revendication 4, qui est le poly[imino[chlorhydrate de 1-[[[[2-[(amino-iminométhyl)-amino]éthyl]amino]carbonyl]méthyl]-2-oxo-1,2-éthanediyle]].

15. Un composé de la revendication 5, qui est le poly[imino[dichlorure de 1-[[[[2-[[3-(triméthylammonio)-propyl]diméthylammonio]éthyl]amino]carbonyl]méthyl]-2-oxo-1,2-éthanediyle]].

16. Un composé de la revendication 5, qui est le poly[imino[dichlorure de 1-[[[[2-[[2-(triméthylammonio)-éthyl]diméthylammonio]éthyl]amino]carbonyl]méthyl]-2-oxo-1,2-éthanediyle]].

17. Un composé de la revendication 5, qui est le poly[imino[chlorure de 1-[[[[2-[1-méthylpyrrolidinium-1-yl]éthyl]amino]carbonyl]méthyl]-2-oxo-1,2-éthanediyle]].

18. Un composé de la revendication 5, qui est le poly[imino[chlorure de 1-[[[[2-[bis(2-hydroxyéthyl)méthyl-ammonio]éthyl]amino]carbonyl]méthyl]-2-oxo-1,2-éthanediyle]].

19. Un composé de la revendication 5, qui est le poly[imino[chlorure de 1-[[[[2-[diméthyl(phénylméthyl)-ammonio]éthyl]amino]carbonyl]méthyl]-2-oxo-1,2-éthanediyle]].

20. Un composé de la revendication 5, qui est le poly[imino[chlorure de 1-[[[[3-[diméthyl(phénylméthyl)-ammonio]propyl]amino]carbonyl]méthyl]-2-oxo-1,2-éthanediyle]].

21. Un copolymère ayant des motifs monomères de la formule suivante [NH-(C₂H₃)A-CO] dans laquelle A est (CH₂)_{w}-CO-HN-(CH₂)_{y}-(CH₂)ₜR-(CH₂)_{z}-Q et dans laquelle
w est le nombre entier 0 ou 1 ;
y est un nombre entier de 1 à 6 ;
z est un nombre entier de 0 à 3 ;
t est le nombre entier 0 ou 1 ;
Q est 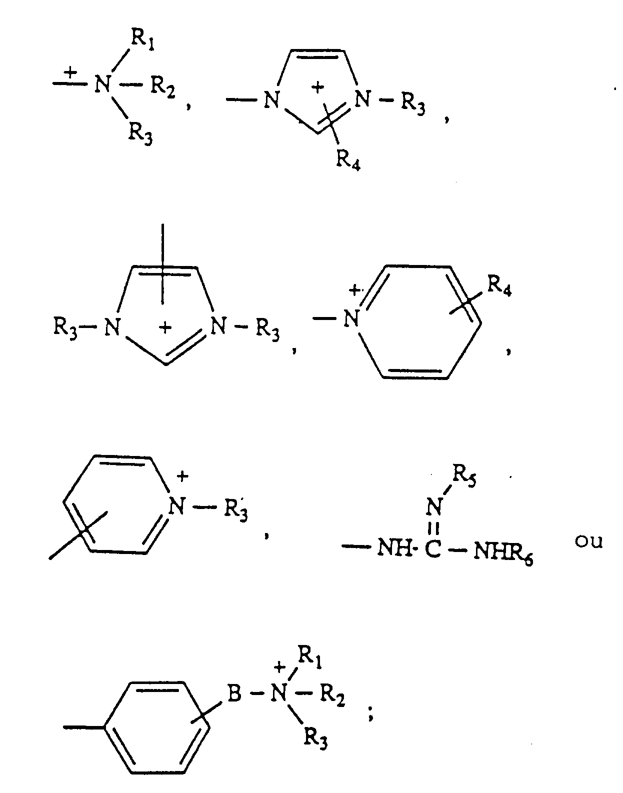
R est l'hydrogène, un groupe alkyle en C₁-C₄ à chaîne droite ou ramifiée, phényle ou, s'il est pris avec R₁, forme un monohétérocycle saturé penta- ou hexagonal ;
R₁, R₂ sont chacun identiquement ou indépendamment un groupe alkyle en C₁-C₁₀ à chaîne droite ou ramifiée qui peut être substitué par un maximum de 3 substituants choisis parmi 1 ou 2 groupes hydroxyle et un groupe 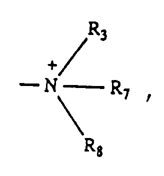 ou bien, s'ils sont pris ensemble, forment un hétérocycle saturé penta- ou hexagonal qui peut contenir une liaison 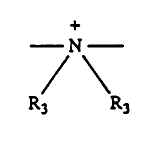 ou -O- ;
R₃ est un groupe méthyle, éthyle, benzyle qui peut être substitué par un maximum de 3 substituants choisis parmi les groupes hydroxyle, halogéno, méthoxy et alkyle en C₁-C₆ à chaîne droite ou ramifiée, 1-naphtyle ou 2-naphtyle ;
R₄ est un groupe alkyle en C₁-C₆ à chaîne droite ou ramifiée qui peut être substitué par un groupe hydroxyle ;
R₅, R₆ sont chacun identiquement ou indépendamment l'hydrogène, un groupe alkyle en C₁-C₄ à chaîne droite ou ramifiée, ou bien, s'ils sont pris ensemble, forment un dihétérocycle penta- ou hexagonal ;
R₇, R₈ sont chacun identiquement ou indépendamment un groupe alkyle en C₁-C₆ à chaîne droite ou ramifiée qui peut être substitué par un groupe hydroxyle, ou bien, s'ils sont pris ensemble avec une liaison -O-, forment un cycle de morpholine ;
B est une liaison directe ou un groupe alkyle en C₁-C₄ à chaîne droite ou ramifiée ;
X⁻ est un anion formateur de sel pharmaceutiquement acceptable ;
et dans lequel ledits motifs monomères sont différents et le nombre total desdits motifs se situe dans l'intervalle d'environ 50 à 500.

22. L'utilisation d'un composé de la formule structurale II suivante : 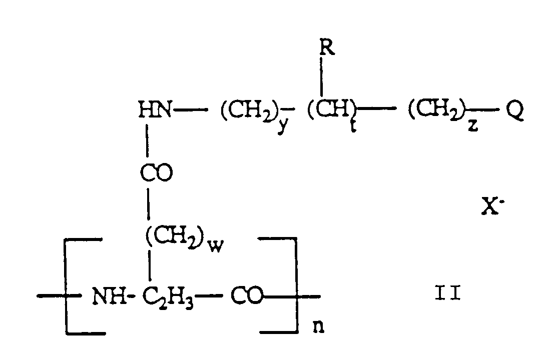
dans laquelle
w est le nombre entier 0 ou 1 ;
y est un nombre entier de 1 à 6 ;
z est un nombre entier de 0 à 3 ;
t est le nombre entier 0 ou 1 ;
Q est 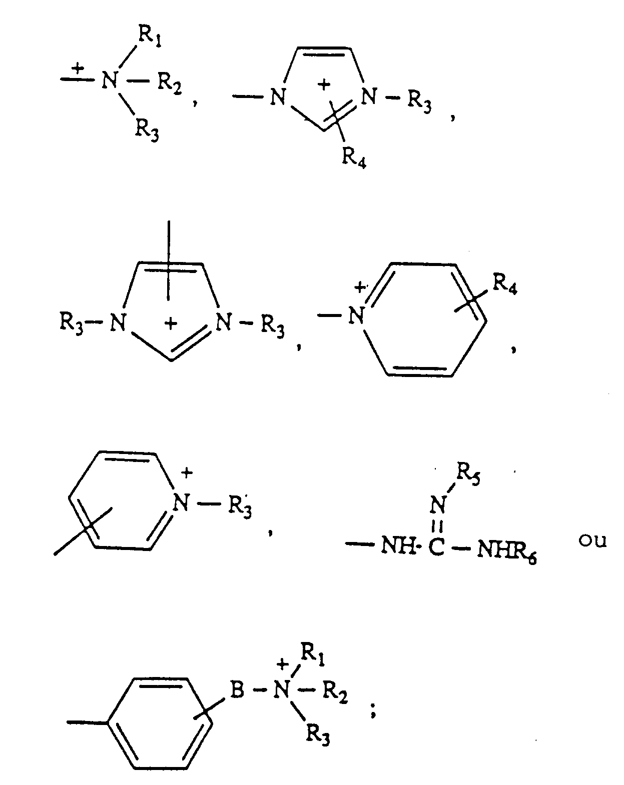
R est l'hydrogène, un groupe alkyle en C₁-C₄ à chaîne droite ou ramifiée, phényle ou, s il est pris avec R₁, forme un monohétérocycle saturé penta- ou hexagonal ;
R₁, R₂ sont chacun identiquement ou indépendamment un groupe alkyle en C₁-C₁₀ à chaîne droite ou ramifiée qui peut être substitué par un maximum de 3 substituants choisis parmi 1 ou 2 groupes hydroxyle et un groupe 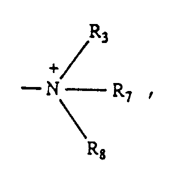 ou bien, s'ils sont pris ensemble, forment un hétérocycle saturé penta- ou hexagonal qui peut contenir une liaison 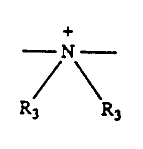 ou -O- ;
R₃ est un groupe méthyle, éthyle, benzyle qui peut être substitué par un maximum de 3 substituants choisis parmi les groupes hydroxyle, halogéno, méthoxy et alkyle en C₁-C₆ à chaîne droite ou ramifiée, 1-naphtyle ou 2-naphtyle ;
R₄ est un groupe alkyle en C₁-C₆ à chaîne droite ou ramifiée qui peut être substitué par un groupe hydroxyle ;
R₅, R₆ sont chacun identiquement ou indépendamment l'hydrogène, un groupe alkyle en C₁-C₄ à chaîne droite ou ramifiée, ou bien, s'ils sont pris ensemble, forment un dihétérocycle penta- ou hexagonal ;
R₇, R₈ sont chacun identiquement ou indépendamment un groupe alkyle en C₁-C₆ à chaîne droite ou ramifiée qui peut être substitué par un groupe hydroxyle, ou bien, s'ils sont pris ensemble avec une liaison -O-, forment un cycle de morpholine ;
B est une liaison directe ou un groupe alkyle en C₁-C₄ à chaîne droite ou ramifiée ;
n est un nombre entier compris dans l'intervalle d'environ 50 à 500 ; et
X⁻ est un anion formateur de sel pharmaceutiquement acceptable,
pour la fabrication d'une composition pharmaceutique destinée à réduire l'activité lipidique chez un mammifère.

23. L'utilisation d'un composé de la formule structurale II suivante : 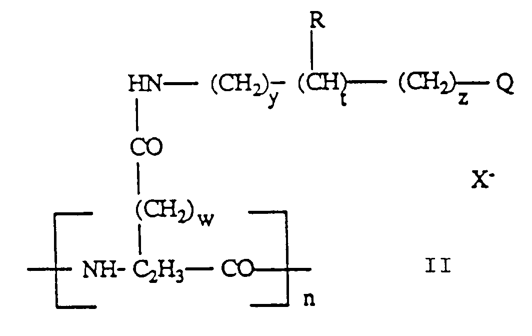 dans laquelle
w est le nombre entier 0 ou 1 ;
y est un nombre entier de 1 à 6 ;
z est un nombre entier de 0 à 3 ;
t est le nombre entier 0 ou 1 ;
Q est 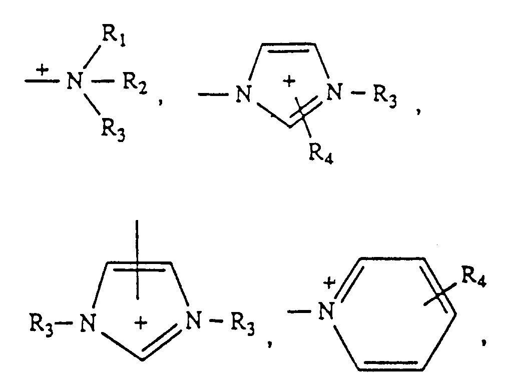 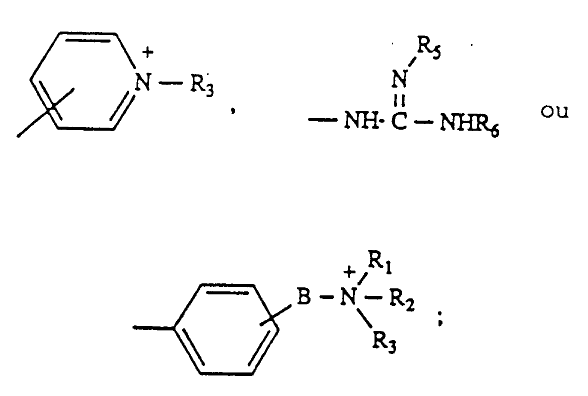
R est l'hydrogène, un groupe alkyle en C₁-C₄ à chaîne droite ou ramifiée, phényle ou, s'il est pris avec R₁, forme un monohétérocycle saturé penta- ou hexagonal ;
R₁, R₂ sont chacun identiquement ou indépendamment un groupe alkyle en C₁-C₁₀ à chaîne droite ou ramifiée qui peut être substitué par un maximum de 3 substituants choisis parmi 1 ou 2 groupes hydroxyle et un groupe 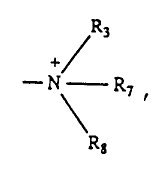 ou bien, s'ils sont pris ensemble, forment un hétérocycle saturé penta- ou hexagonal qui peut contenir une liaison 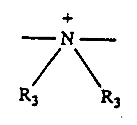 ou -O- ;
R₃ est un groupe méthyle, éthyle, benzyle qui peut être substitué par un maximum de 3 substituants choisis parmi les groupes hydroxyle, halogéno, méthoxy et alkyle en C₁-C₆ à chaîne droite ou ramifiée, 1-naphtyle ou 2-naphtyle ;
R₄ est un groupe alkyle en C₁-C₆ à chaîne droite ou ramifiée qui peut être substitué par un groupe hydroxyle ;
R₅, R₆ sont chacun identiquement ou indépendamment l'hydrogène, un groupe alkyle en C₁-C₄ à chaîne droite ou ramifiée, ou bien, s'ils sont pris ensemble, forment un dihétérocycle penta- ou hexagonal ;
R₇, R₈ sont chacun identiquement ou indépendamment un groupe alkyle en C₁-C₆ à chaîne droite ou ramifiée qui peut être substitué par un groupe hydroxyle, ou bien, s'ils sont pris ensemble avec une liaison -O-, forment un cycle de morpholine ;
B est une liaison directe ou un groupe alkyle en C₁-C₄ à chaîne droite ou ramifiée ;
n est un nombre entier compris dans l'intervalle d'environ 50 à 500 ; et
X⁻ est un anion formateur de sel pharmaceutiquement acceptable,
pour la fabrication d'une composition pharmaceutique destinée à dissoudre, retarder la progression et/ou bloquer la formation des calculs biliaires chez l'homme.

24. Une composition pharmaceutique comprenant une quantité, efficace pour abaisser le taux de lipides, d'un composé de la Formule II suivante : 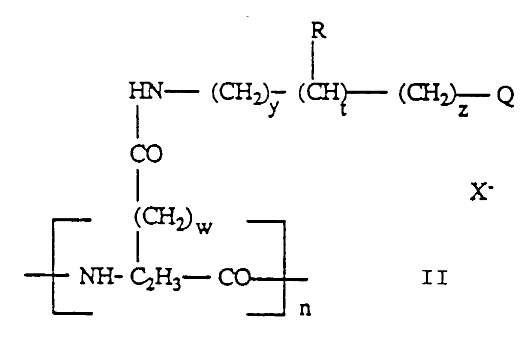 dans laquelle
w est le nombre entier 0 ou 1 ;
y est un nombre entier de 1 à 6 ;
z est un nombre entier de 0 à 3 ;
t est le nombre entier 0 ou 1 ;
Q est 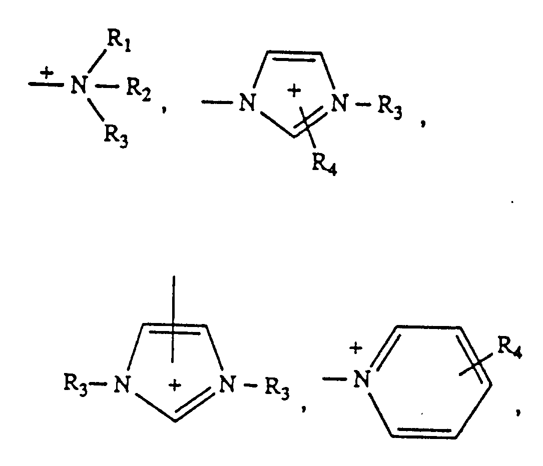 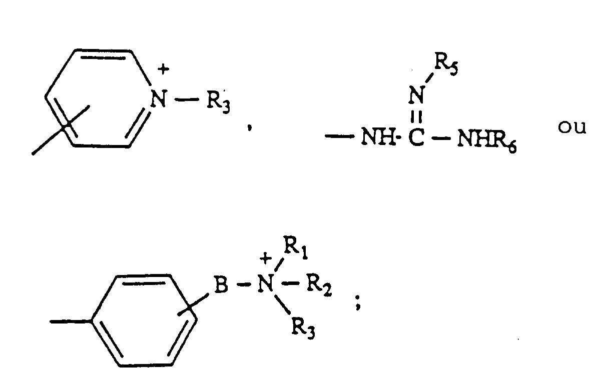
R est l'hydrogène, un groupe alkyle en C₁-C₄ à chaîne droite ou ramifiée, phényle ou, s'il est pris avec R₁, forme un monohétérocycle saturé penta- ou hexagonal ;
R₁, R₂ sont chacun identiquement ou indépendamment un groupe alkyle en C₁-C₁₀ à chaîne droite ou ramifiée qui peut être substitué par un maximum de 3 substituants choisis parmi 1 ou 2 groupes hydroxyle et un groupe 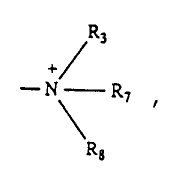 ou bien, s'ils sont pris ensemble, forment un hétérocycle saturé penta- ou hexagonal qui peut contenir une liaison 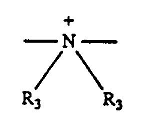 ou -O- ;
R₃ est un groupe méthyle, éthyle, benzyle qui peut être substitué par un maximum de 3 substituants choisis parmi les groupes hydroxyle, halogéno, méthoxy et alkyle en C₁-C₆ à chaîne droite ou ramifiée, 1-naphtyle ou 2-naphtyle ;
R₄ est un groupe alkyle en C₁-C₆ à chaîne droite ou ramifiée qui peut être substitué par un groupe hydroxyle ;
R₅, R₆ sont chacun identiquement ou indépendamment l'hydrogène, un groupe alkyle en C₁-C₄ à chaîne droite ou ramifiée, ou bien, s'ils sont pris ensemble, forment un dihétérocycle penta- ou hexagonal ;
R₇, R₈ sont chacun identiquement ou indépendamment un groupe alkyle en C₁-C₆ à chaîne droite ou ramifiée qui peut être substitué par un groupe hydroxyle, ou bien, s'ils sont pris ensemble avec une liaison -O-, forment un cycle de morpholine ;
B est une liaison directe ou un groupe alkyle en C₁-C₄ à chaîne droite ou ramifiée ;
n est un nombre entier compris dans l'intervalle d'environ 50 à 500 ; et
X⁻ est un anion formateur de sel pharmaceutiquement acceptable,
ainsi qu'un support non toxique pharmaceutiquement acceptable.
